# EUROPEAN PATENT APPLICATION

(11) **EP 2 324 887 A1**
(43) Date of publication of application: **25.05.2011**
(21) Application number: 10014088.8
(22) Date of filing: 21.10.2003
(51) Int. Cl.: A61Q 15/00, A61K 8/26, A61K 8/86

(54) **Deodorizing skin treatment composition**

(30) Priority: 25.10.2002 JP 2002311033; 04.04.2003 JP 2003101489; 25.09.2003 JP 2003332802
(62) Divisional of application: 03756710.4
(71) Applicant: Shiseido Company, Limited, Tokyo 104-8010 (JP)
(72) Inventor: Nakane, Toshihiko, Yokohama-shi Kanagawa 224-8558 (JP); Ishino, Hirokazu, Yokohama-shi Kanagawa 224-8558 (JP); Isa, Takashi, Yokohama-shi Kanagawa 227-0038 (JP); Oguchi, Nozomi, Yokohama-shi Kanagawa 224-8558 (JP); Tominaga, Naoki, Yokohama-shi Kanagawa 224-8558 (JP); Kamiya, Yukiko, Yokohama-shi Kanagawa 224-8558 (JP)
(74) Representative: Hollatz, Christian

(57) **Abstract**

A skin treatment composition comprising anti-bacterial zeolite and polyoxyethylene polyoxypropylene 2-decyltetradecyl ether.
The object is to provide a skin treatment composition containing anti-bacterial zeolite that exhibits the effect of preventing discoloration of the skin treatment compositions and the effect of preventing the skin treatment composition from staining clothing and/or reducing the degree of such staining.

## Description

### TECHNICAL FIELD

The present invention relates to a skin treatment composition. The skin treatment composition of the present invention is preferably used as a deodorizing skin treatment composition, deodorizing cosmetic, antiperspirant cosmetic, odor eliminating cosmetic, etc., mainly for the purpose of deodorization.
(1) More specifically, the present invention relates to a skin treatment composition containing anti-bacterial zeolite that has superior anti-discoloring properties.
(2) Even more specifically, it relates to a skin treatment composition that is a deodorizing cosmetic containing anti-bacterial zeolite and also is superior in formulation stability such as anti-discoloring properties and dispersibility of powder components, as well as very superior in terms of tactile sensation during use.
(3) More specifically, the present invention relates to a skin treatment composition containing anti-bacterial zeolite that has superior anti-staining properties.

### BACKGROUND ART

### [Invention of claim 1]

Anti-bacterial zeolite is blended into skin treatment compositions including cosmetics and quasi-drugs as a preservative and/or odor eliminating agent.

For example, a composition for anti-bacterial sprays (see Patent Document 1-1) and deodorizing cosmetics (see Patent Document 1-2) containing anti-bacterial zeolite have been developed. Also, technology that blends silicone into anti-bacterial zeolite as a deodorizing cosmetic with improved anti-discoloring properties has been disclosed (see Patent Document 1-3).
Patent Document 1-1: Japanese Patent Laid-Open No. Sho 63-250325 bulletin
Patent Document 1-2: Japanese Patent Laid-Open No. Hei 8-26956 bulletin
Patent Document 1-3: Japanese Patent Laid-Open No. Hei 8-92051 bulletin

Anti-bacterial zeolite by itself is a stable ingredient for a skin treatment composition. However, blending anti-bacterial zeolite into a skin treatment composition sometimes causes discoloration.

The cause of this discoloration is not clear; there are many raw materials in a skin treatment composition and reactions with such raw materials or their impurities are believed to cause subtle discoloration.

For example, the inventors verified that blending anti-bacterial zeolite as a preservative or odor eliminating agent into an antiperspirant cosmetic containing chlorhydroxy aluminum causes discoloration that is not preferable for the cosmetic's appearance. Also, the inventors verified that blending anti-bacterial zeolite in a skin treatment composition containing various surfactants results in undesirable discoloration.

In view of the aforementioned problem, the inventors conducted earnest research and amazingly discovered that trisalt ethylenediaminehydroxyethyl triacetate has an anti-discoloration effect on anti-bacterial zeolite in skin treatment compositions and thus completed the present invention.

The object of the present invention is to provide a skin treatment composition containing anti-bacterial zeolite that has the superb effect of preventing discoloration of skin treatment compositions and/or reducing the degree of discoloration.

### [Invention of claims 2-6]

Anti-bacterial zeolite powder is blended into skin treatment compositions including cosmetics and quasi-drugs as a preservative and/or odor eliminating agent.

For example, a composition for anti-bacterial sprays (see Patent Document 2-1) and deodorizing cosmetics (see Patent Document 2-2) containing anti-bacterial zeolite have been developed. Also, technology that blends silicone into anti-bacterial zeolite as a deodorizing cosmetic with improved anti-discoloring properties has been disclosed (see Patent Document 2-3).

A disposable sheet-shaped cosmetic has been developed as an antiperspirant cosmetic containing alum (see Patent Document 2-4).
Patent Document 2-1: Japanese Patent Laid-Open No. Sho 63-250325 bulletin
Patent Document 2-2: Japanese Patent Laid-Open No. Hei 8-26956 bulletin
Patent Document 2-3: Japanese Patent Laid-Open No. Hei 8-92051 bulletin
Patent Document 2-4: Japanese Patent Laid-Open No. 2001-114660 bulletin

A deodorizing cosmetic is a cosmetic that is used to prevent or control emanation and/or secretion of offensive body odor, or to eliminate the emanated and/or secreted components. In terms of the product form, it is commonly used as a lotion, cream, powder, stick, aerosol, etc.

Body odor is odor caused by decomposition of perspiration. The following methods are available for preventing body odor arising with perspiration.

### (1) Deodorizing method utilizing astringent actions

This method indirectly prevents body odor by supressing perspiration through a strong astringent action. For example, astringent agents such as zinc sulfocarbolate, citric acid, and various aluminum compounds are frequently used. Ethyl alcohol has an astringent action, too. Among them, an aluminum compound (chlorhydroxy aluminum) is used particularly frequently; for the aerosol type products, a complex with propylene glycol, which has superior compatibility with freon gas, has been developed.

### (2) Deodorizing method utilizing bactericidal actions

Perspiration is decomposed and gives rise to odor due to the decomposing actions of bacteria. Therefore, a bactericide can be used to prevent the growth of bacteria and thus directly prevent decomposition of perspiration and offensive odor. For example, TMTD (tetramethyl thiuram disulfide), benzalconium chloride, halocalban, etc. are commonly used. In addition, zinc flower, essential oil, perfume, chlorophyll compounds, etc. also have an anti-bacterial action and exhibit a deodorizing effect.

### (3) Deodorizing method utilizing masking actions

Normal body odor can be masked by perfume and/or cologne to eliminate the smell. Also, there are methods that blend the aforementioned bactercide in the perfume and/or cologne to promote the deodorizing effect.

The deodorizing cosmetics disclosed in Patent Documents 2-1 to 2-3 use a deodorizing method utilizing the bactericidal action of anti-bacterial zeolite. However, there was a problem in that a deodorizing cosmetic using anti-bacterial zeolite is discolored and the product stability is difficult to maintain when an antiperspirant containing a halogen such as chlorhydroxy aluminum is used. Furthermore, there was a problem in terms of usability because it does not feel smooth on the skin. Therefore, development of a deodorizing cosmetic that has superior formulation stability, superior usability, and a good deodorizing effect is desired.

The object of the present invention is to provide a skin treatment composition that is superior in terms of the deodorizing effect in addition to formulation stability and usability.

In order to solve the aforementioned problem in view of the description above, the inventors conducted earnest research on the causes of discoloration and poor usability of conventional deodorizing cosmetics and discovered that the addition of a halogen compound such as chlorhydroxy aluminum, used as an antiperspirant, causes discoloration of the formulation and also causes inhomogeneity in the formulation, which leads to aggregation, causing a granular texture, resulting in poor usability. The inventors also discovered that the addition of alum or dried alum, instead of a halogen compound such as chlorhydroxy aluminum, improves the formulation stability, elminates the granular texture at the time of use, and gives a superior deodorizing effect, and thus completed the present invention.

Conventional deodorizing cosmetics containing anti-bacterial zeolite do not have satisfactory formulation stability in terms of dispersibility and discoloration when a large amount of a halogen-containing compound such as chlorhydroxy aluminum is added. It is believed that the anti-bacterial metal in the anti-bacterial zeolite interacts with the antiperspirant such as chlorhydroxy aluminum to cause discoloration. The most important point of the present invention is the discovery of the fact that superior formulation stability and a superior tactile sensation on the skin during the use are achieved when alum or dried alum, which acts as an antiperspirant, is added in addition to anti-bacterial zeolite, which could never be predicted from conventional technology. In the present invention, an antiperspirant containing a halogen such as chlorhydroxy aluminum can be added as long as the blend ratio is within the range that virtually does not affect the product in terms of its dispersibility or discoloration, compared with the blend ratios of anti-bacterial zeolite and alum and/or dried alum. In that case, the blend ratio of the antiperspirant containing a halogen is preferably 5 mass % or less of the total amount of the skin treatment composition.

The reason why the tactile sensation during the use is superior in the present invention is believed to be stabilization of the formulation due to the improvement in the dispersibility; however the mechanism of action is not clear.

### [Invention of claims 7-8]

Anti-bacterial zeolite is blended into skin treatment compositions including cosmetics and quasi-drugs as a preservative and/or odor eliminating agent.

For example, a composition for anti-bacterial sprays (see Patent Document 3-1) and deodorizing cosmetics (see Patent Document 3-2) containing anti-bacterial zeolite have been developed. Also, technology that blends silicone into anti-bacterial zeolite as a deodorizing cosmetic with improved anti-discoloring properties has been disclosed (see Patent Document 3-3).

However, there has been no report on skin treatment compositions and/or cosmetics with improved anti-staining properties.

On the other hand, polyoxyethylene polyoxypropylene 2-decyltetradecyl ether is publicly known as a surfactant to be blended into cosmetics (see Patent Document 3-4).
Patent Document 3-1: Japanese Patent Laid-Open No. Sho 63-250325 bulletin
Patent Document 3-2: Japanese Patent Laid-Open No. Hei 8-26956 bulletin
Patent Document 3-3: Japanese Patent Laid-Open No. Hei 8-92051 bulletin
Patent Document 3-4: Japanese Patent No. 3323339 bulletin

Anti-bacterial zeolite by itself is a stable ingredient for a skin treatment composition.

However, when anti-bacterial zeolite is blended into a skin treatment composition, discoloration occurs and clothing can be stained if this adheres to the clothing.

The cause of this discoloration is not clear; there are many raw materials in a skin treatment composition and reactions with such raw materials or their impurities are believed to cause subtle discoloration.

For example, the inventors verified that blending anti-bacterial zeolite as a preservative or odor eliminating agent in an antiperspirant cosmetic containing chlorhydroxy aluminum causes discoloration that is not preferable for the cosmetic's appearance.

The inventors also verified that perspiration and sunlight can cause undesirable discoloration in an antiperspirant cosmetic and clothing is stained if this adheres to it.

In view of the aforementioned problem, the inventors conducted earnest research and amazingly discovered that anti-bacterial zeolite and polyoxyethylene polyoxypropylene 2-decyltetradecyl ether, when used together, have an anti-staining effect and thus completed the present invention.

The object of the present invention is to provide a skin treatment composition containing anti-bacterial zeolite that has the superb effect of preventing the staining of clothes.

### DISCLOSURE OF INVENTION

### [Invention of claim 1]

That is, the present invention provides a skin treatment composition comprising anti-bacterial zeolite and trisalt ethylenediaminehydroxyethyl triacetate.

### [Invention of claims 2-6]

That is, the present invention provides a skin treatment composition comprising anti-bacterial zeolite and alum and/or dried alum.

Also, the present invention provides the aforementioned skin treatment composition wherein the content of said anti-bacterial zeolite is 0.1-90 mass % of the total amount of the skin treatment composition.

Furthermore, the present invention provides the aforementioned skin treatment composition wherein the content of said alum and/or dried alum is 0. 1 or more in terms of the mass ratio to said anti-bacterial zeolite.

Also, the present invention provides the aforementioned skin treatment composition wherein the content of said anti-bacterial zeolite is 0.1-70 mass % of the total amount of the deodorizing cosmetic and the content of said alum and/or dried alum is 0.01-80 mass % of the total amount of the skin treatment composition.

Furthermore, the present invention provides the aforementioned skin treatment composition wherein the average particle size of said anti-bacterial zeolite is 10 micrometers or less, the particle size distribution is such that 20% or less of the particles have a particle size larger than 15 micrometers, and the average particle size of said alum and/or dried alum is 0.01-50 micrometers.

### [Invention of claims 7-8]

That is, the present invention provides a skin treatment composition comprising anti-bacterial zeolite and polyoxyethylene polyoxypropylene 2-decyltetradecyl ether.

Also, the present invention provides the aforementioned skin treatment composition wherein the polyoxyethylene unit of the polyoxyethylene polyoxypropylene 2-decyltetradecyl ether is 20-28 E.0. and the polyoxypropylene unit is 10-16 P.0.

### BEST MODE FOR CARRYING OUT THE INVENTION

The present invention is described in detail below.

### [Invention of claim 1]

The anti-bacterial zeolite used in the present invention is zeolite that holds anti-bacterial metal ions in its ion-exchangeable parts; i.e. zeolite whose exchangeable ions are partly or entirely replaced by an anti-bacterial metal. In the present invention, zeolite having ammonium ion substitution in addition to anti-bacterial metal ion substitution is also preferable.

For the zeolite, either natural zeolite or synthetic zeolite can be used. Zeolite is aluminosilicate having a three dimensional skeletal structure; it is represented by the general formula XM_{2/n} 0·Al₂0₃ · YSi0₂ · ZH₂0. In this general formula, M denotes an exchangeable ion, usually a monovalent or divalent metal ion. n denotes the atomic valence of the (metal) ion. X and Y denote metal oxide and the silica factor, respectively, and Z denotes the number of the crystallization water molecules.

Specific examples of zeolite include A-type zeolite, X-type zeolite, Y-zeolite, T-type, high silica zeolite, sodalite, mordenite, analcime, crinoptyrolite, chabasite, and erionite. The ion exchange capacity of these zeolites are: 7 meq/g for A-type zeolite, 6.4 meq/g for X-type zeolite, 5 meq/g for Y-zeolite, 3.4 meq/g for T-type, 11.5 meq/g for sodalite, 2.6 meq/g for mordenite, 5 meq/g for analcime, 2.6 meq/g for crinoptyrolite, 5 meq/g for chabasite, and 3. 8 meq/g for erionite. Any of these has enough capacity for ion exchange with anti-bacterial metal ions and/or ammonium ions.

Examples of exchangeable ions in zeolite include sodium ions, calcium ions, potassium ions, magnesium ions, and iron ions. Examples of the anti-bacterial metal ions to use to substitute these ions include silver, copper, zinc, mercury, tin, lead, bismuth, cadmium, chromium, and thallium ions; preferably silver, copper, or zinc ions, and more preferably silver ions.

In terms of anti-bacterial action, the content of the anti-bacterial metal ions is preferably 0.1-15 mass % in the zeolite. For example, anti-bacterial zeolite containing 0.1-15% of silver ions and 0.1-8 mass % of copper ions or zinc ions is preferable. On the other hand, zeolite can contain up to 20 mass % of ammonium ions; however, for the purpose of effectively preventing discoloration of the zeolite, 0.5-5% is preferable and 0.5-2 mass % is more preferable. "Mass %" means the mass percentage in 110°C dry standard zeolite.

In the present invention, commercial products can be used for the anti-bacterial zeolite; the anti-bacterial zeolite can also be prepared by the following method, for example. That is, zeolite is exposed to a mixed solution containing anti-bacterial metal ions such as silver ions, copper ions, and zinc ions, prepared in advance, to substitute the aforementioned ions for the exchangeable ions in the zeolite. The exposure can be achieved by the batch method or continuous method (column method, for example) for 3-24 hours, preferably 10-24 hours, at 10-70°C, preferably 40-60°C. The pH of the aforementioned mixed solution should be adjusted to 3-10, preferably 5-7. This adjustment is preferable because precipitation of silver oxide and such on the surface or in the pores of the zeolite can be prevented by this. Each ion in the mixed aqueous solution is usually supplied in the form of a salt. For example, silver ions are from silver nitrate, silver sulfate, silver perchlorate, diamminesilver nitrate, diamminesilver sulfate, etc.; copper ions are from copper nitrate (II), copper perchlorate, copper acetate, potassium tetracyanocuprate, copper sulfate, etc.; zinc ions are from zinc nitrate (II), zinc sulfate, zinc perchlorate, zinc thiocyanate, zinc acetate, etc. ; mercury ions are from mercury perchlorate, mercury nitrate, and mercury acetate; tin ions are from tin sulfate and such; lead ions are from lead sulfate, lead nitrate, etc. : bismuth ions are from bismuth chloride, bismuth iodide, etc. ; cadmium ions are from cadmium perchlorate, cadmium sulfate, cadmium nitrate, and cadmium acetate; chromium ions are from chromium perchlorate, chromium sulfate, chromium ammonium sulfate, chromium nitrate, etc.; thallium ions are from thallium perchlorate, thallium sulfate, thallium nitrate, thallium acetate, etc.

The anti-bacterial metal ion content in the zeolite can be controlled by adjusting the concentration of each ion (salt) in said mixed aqueous solution. For example, in the case of anti-bacterial zeolite containing silver ions, an anti-bacterial zeolite with a silver ion content of 0.1-5% can be obtained by adjusting the silver ion concentration in said mixed aqueous solution to 0. 002M/1-0. 15M/1. In the case of anti-bacterial zeolite additionally containing copper ions and zinc ions, an anti-bacterial zeolite with a copper ion content of 0.1-8% and a zinc ion content of 0. 1-8% can be obtained by adjusting the silver ion concentration to 0.1M/1-0.85M/1 and the zinc ion concentration to 0.15M/1-1.2M/1 in said mixed aqueous solution. For ion exchange of anti-bacterial zeolite, it is also possible to use solutions each of which contains each ion and expose the zeolite with these solutions one after another. The concentration of each ion in each aqueous solution can be determined based on the concentration of each ion in said mixed aqueous solution.

After the completion of the ion exchange, the zeolite is thoroughly rinsed and then dried. The drying is preferably conducted at 105°C-115°C, or under a reduced pressure (1-30 Torr) at 70-90°C.

Ion exchange for organic ions and/or for ions for which there isn't adequate water soluble salts, such as tin and bismuth, can be done by using an organic solvent solution such as alcohol and acetone to prevent precipitation of slightly soluble basic salts.

The blend ratio of the anti-bacterial zeolite in the skin treatment composition is not limited in particular. It is determined based on the reason why the anti-bacterial zeolite is added and also on the product form of the skin treatment composition.

For example, when blended in as a preservative, the blend ratio is usually 0. 05-10 mass % of the total amount of the skin treatment composition. As another example, when blended in as a bactericide, the blend ratio is usually 0.1-90 mass % of the total amount of the skin treatment composition, depending on the product form. For example, for lotion or cream type skin treatment compositions 0. 1-20 mass % of the total amount of the skin treatment composition is preferable; for powder type skin treatment compositions 0. 5-80 mass % of the total amount of the skin treatment composition is preferable; for stick type skin treatment compositions 0. 5-60 mass % of the total amount of the skin treatment composition is preferable; and for spray type skin treatment compositions 0. 5-50 mass % of the total amount of the skin treatment composition is preferable.

The trisalt ethylenediaminehydroxyethyl triacetate used in the present invention is a prior art skin treatment composition ingredient used as a chelating agent. Examples of the salt include alkali metal salts such as sodium and potassium; sodium salt is preferable. Commercial products such as Clewat 0H-300 (Teikoku Kagaku Sangyo Co., Ltd.) are used. Dry powder of trisalt ethylenediaminehydroxyethyl triacetate is blended into the skin treatment composition of the present invention usually in the form of trihydrate.

In the present invention, trisalt ethylenediaminehydroxyethyl triacetate specifically acts as an anti-discoloration agent for a skin treatment composition containing anti-bacterial zeolite. EDTA-3Na and such, which are well known as a chelating agent and have a chemical structure similar to that of trisalt ethylenediaminehydroxyethyl triacetat such as trisodium ethylenediaminehydroxyethyl triacetate, do not have the anti-discoloration effect for skin treatment compositions.

The blend ratio of the trisalt ethylenediaminehydroxyethyl triacetate is determined based on the blend ratio of the anti-bacterial zeolite and the product form. It is usually 0.01-5 mass % (unhydrated equivalent) of the total amount of the skin treatment composition.

When using the skin treatment composition of the present invention for applications such as odor eliminating agents and antiperspirant cosmetics, it is preferable to blend in an aluminum compound that is an antiperspirant.

In addition to the aforementioned essential ingredients, other ingredients commonly used in skin treatment compositions, for example one, two or more of those listed below, are blended in as necessary in the skin treatment composition of the present invention; the preparation can be conducted for the target formulation with a conventional method.

### [Invention of claims 2-6]

The anti-bacterial zeolite used in the present invention is zeolite powder that holds anti-bacterial metal ions in its ion-exchangeable parts; i.e. zeolite powder whose exchangeable ions are partly or entirely replaced by anti-bacterial metal. In the present invention, zeolite having ammonium ion substitution in addition to anti-bacterial metal ion substitution is also preferable.

For the zeolite, either natural zeolite or synthetic zeolite can be used. Zeolite is aluminosilicate having a three dimensional skeletal structure; it is represented by the general formula XM_{2/n}0·Al₂0₃·YSi0₂·ZH₂0. In this general formula, M denotes an exchangeable ion, usually a monovalent or divalent metal ion. n denotes the atomic valence of the (metal) ion. X and Y denote metal oxide and the silica factor, respectively, and Z denotes the number of the crystallization water molecules.

Specific examples of zeolite include A-type zeolite, X-type zeolite, Y-zeolite, T-type, high silica zeolite, sodalite, mordenite, analcime, crinoptyrolite, chabasite, and erionite. The ion exchange capacity of these zeolites are: 7 meq/g for A-type zeolite, 6. 4 meq/g for X-type zeolite, 5 meq/g for Y-zeolite, 3.4 meq/g for T-type, 11.5 meq/g for sodalite, 2.6 meq/g for mordenite, 5 meq/g for analcime, 2.6 meq/g for crinoptyrolite, 5 meq/g for chabasite, and 3. 8 meq/g for erionite. Any of these has enough capacity for ion exchange with anti-bacterial metal ions and/or ammonium ions.

Examples of exchangeable ions in zeolite include sodium ions, calcium ions, potassium ions, magnesium ions, and iron ions. Examples of the anti-bacterial metal ions to use to substitute these ions include silver, copper, zinc, mercury, tin, lead, bismuth, cadmium, chromium, and thallium ions; preferably silver, copper, or zinc ions, and more preferably silver ions.

The content of the anti-bacterial metal ions is preferably 0. 1-15 mass % of the zeolite. For example, anti-bacterial zeolite containing 0.1-15% of silver ions and 0.1-8 mass % of copper ions or zinc ions is preferable. On the other hand, zeolite can contain up to 20 mass % of ammonium ions; however, for the purpose of effectively preventing discoloration of the zeolite, 0.5-5 mass % is preferable and 0.5-2 mass % is more preferable. "Mass %" means the mass percentage in 110°C dry standard zeolite.

In the present invention, commercial products can be used for the anti-bacterial zeolite. The anti-bacterial zeolite is prepared, for example, as follows. That is, zeolite is exposed to a mixed solution containing anti-bacterial metal ions such as silver ions, copper ions, and zinc ions, prepared in advance, to substitute the aforementioned ions for the exchangeable ions in the zeolite. The exposure can be achieved by the batch method or continuous method (column method, for example) for 3-24 hours, preferably 10-24 hours, at 10-70°C, preferably 40-60°C. The pH of the aforementioned mixed solution should be adjusted to 3-10, preferably 5-7. This adjustment is preferable because precipitation of silver oxide and such on the surface or in the pores of the zeolite can be prevented by this. Each ion in the mixed aqueous solution is usually supplied in the form of a salt. For example, silver ions are from silver nitrate, silver sulfate, silver perchlorate, diamminesilver nitrate, diamminesilver sulfate, etc.; copper ions are from copper nitrate (II), copper perchlorate, copper acetate, potassium tetracyanocuprate, copper sulfate, etc.; zinc ions are from zinc nitrate (II), zinc sulfate, zinc perchlorate, zinc thiocyanate, zinc acetate, etc. mercury ions are from mercury perchlorate, mercury nitrate, and mercury acetate; tin ions are from tin sulfate and such; lead ions are from lead sulfate, lead nitrate, etc. bismuth ions are from bismuth chloride, bismuth iodide, etc cadmium ions are from cadmium perchlorate, cadmium sulfate, cadmium nitrate, and cadmium acetate; chromium ions are from chromium perchlorate, chromium sulfate, chromium ammonium sulfate, chromium nitrate, etc.; thallium ions are from thallium perchlorate, thallium sulfate, thallium nitrate, thallium acetate, etc.

The anti-bacterial metal ion content in the zeolite can be controlled by adjusting the concentration of each ion (salt) in said mixed aqueous solution. For example, in the case of anti-bacterial zeolite containing silver ions, an anti-bacterial zeolite with a silver ion content of 0.1-5% can be obtained by adjusting the silver ion concentration in said mixed aqueous solution to 0. 002M/1-0. 15M/1. In the case of anti-bacterial zeolite additionally containing copper ions and zinc ions, an anti-bacterial zeolite with a copper ion content of 0. 1-8% and a zinc ion content of 0. 1-8% can be obtained by adjusting the silver ion concentration to 0.1M/1-0.85M/1 and the zinc ion concentration to 0.15M/1-1.2M/1 in said mixed aqueous solution. For ion exchange of anti-bacterial zeolite, it is also possible to use solutions, each of which contains each ion, and expose the zeolite to these solutions one after another. The concentration of each ion in each aqueous solution can be determined based on the concentration of each ion in said mixed aqueous solution.

After the completion of the ion exchange, the zeolite is thoroughly rinsed and then dried. The drying is preferably conducted at 105°C-115°C, or under a reduced pressure (1-30 Torr) at 70-90°C.

Ion exchange for organic ions and/or for ions for which there isn't an adequate water soluble salt, such as tin and bismuth, can be done by using an organic solvent solution such as an alcohol or acetone to prevent precipitation of slightly soluble basic salts.

The blend ratio of the anti-bacterial zeolite is not limited in particular. It is determined based on the product form of the deodorizing cosmetic. Usually, 0.1-90 mass %, preferably 1-70 mass %, more preferably 5-70 mass % of the total amount of the deodorizing cosmetic is blended in depending on the product form.

For the alum and/or dried alum used in the present invention, commercially available powder is used. Examples of preferably used commercial products include Taiace S150, Taiace S100, Taiace K150, and Taiace K20 (TAIMEI Chemicals Co., Ltd.).

The blend ratio of the alum and/or dried alum is not limited in particular. It is determined based on the product form of the skin treatment composition. Usually, 0.1-90 mass %, preferably 1-80 mass %, more preferably 5-70 mass % of the total amount of the skin treatment composition is blended in depending on the product form.

The alum and/or dried alum content is preferably 0.1 or more in terms of the mass ratio with the anti-bacterial zeolite content. When the product form is the aerosol spray type, it is preferable to have 0.1-80 mass % of the anti-bacterial zeolite and 0.1-80 mass % of the alum and/or dried alum, more preferably 0. 5-70 mass % each. When the product form is the stick type, it is preferable to have 0.1-70 mass % of the anti-bacterial zeolite and 0. 1-70 mass % of the alum and/or dried alum, more preferably 0. 5-60 mass % each. When the product form is the powder type, it is preferable to have 0.1-99.9 mass % of the anti-bacterial zeolite and 0.1-99.9 mass % of the alum and/or dried alum, more preferably 50-90 mass % each. When the product form is the lotion type, it is preferable to have 0. 1-30 mass % of the anti-bacterial zeolite and 0. 1-30 mass % of the alum and/or dried alum, more preferably 0.5-20 mass % each.

The average particle size of said anti-bacterial zeolite is preferably 10 micrometers or less. More preferably it is 0.1-5 micrometers. When the average particle size is in this range, it is preferable that 20% or less have a particle size larger than 1 micrometer in terms of the particle size distribution.

Said alum and/or dried alum is preferably fine particle powder having an average particle size of 0.01-50 micrometers.

In addition to the aforementioned essential ingredients, other ingredients commonly used in skin treatment compositions, for example one, two or more of those listed below, are blended as necessary into the skin treatment composition of the present invention; the preparation can be conducted for the target formulation with a conventional method. Preferable products are antiperspirant cosmetics and deodorizing cosmetics that are deodorizing skin treatment compositions.

The product form of the skin treatment composition of the present invention is not limited in particular. Examples include the spray type, roll-on type, powder type and pressed powder type, and stick type. The spray type is prepared by filling a spray container such as an aerosol can or dispenser with the ingredients as well as a propellant such as a liquefied gas and alcohol by using a conventional method. The roll-on type is prepared by filling a roll-on container with the ingredients and alcohol by using a conventional method. For the powder type and the pressed powder type, the ingredients are mixed together with powder components and oil components, and in the case of the powder type the mixture is used as is, and in the case of the pressed powder type the mixture is molded by various molding devices using a conventional method. The stick type is prepared by mixing the ingredients with oil components (solid oil and liquid oil) and filling a container with the mixture, followed by molding, using a conventional method.

### [Invention of claims 7-8]

The anti-bacterial zeolite used in the present invention is zeolite that holds anti-bacterial metal ions in its ion-exchangeable parts.
i. e. zeolite whose exchangeable ions are partly or entirely replaced by anti-bacterial metal ions. In the present invention, zeolite having ammonium ion substitution in addition to anti-bacterial metal ion substitution is also preferable.

For the zeolite, either natural zeolite or synthetic zeolite can be used. Zeolite is aluminosilicate having a three dimensional skeletal structure; it is represented by the general formula XM_{2/n}0.Al₂0₃.YSi0₂.ZH₂0. In this general formula, M denotes an exchangeable ion, usually a monovalent or divalent metal ion. n denotes the atomic valence of the (metal) ion. X and Y denote metal oxide and the silica factor, respectively, and Z denotes the number of the crystallization water molecules.

Specific examples of zeolite include A-type zeolite, X-type zeolite, Y-zeolite, T-type, high silica zeolite, sodalite, mordenite, analcime, crinoptyrolite, chabasite, and erionite. The ion exchange capacity of these zeolites are: 7 meq/g for A-type zeolite, 6.4 meq/g for X-type zeolite, 5 meq/g for Y-zeolite, 3.4 meq/g for T-type, 11. 5 meq/g for sodalite, 2.6 meq/g for mordenite, 5 meq/g for analcime, 2.6 meq/g for crinoptyrolite, 5 meq/g for chabasite, and 3. 8 meq/g for erionite. Any of these has enough capacity for ion exchange with anti-bacterial metal ions and/or ammonium ions.

Examples of exchangeable ions in zeolite include sodium ions, calcium ions, potassium ions, magnesium ions, and iron ions.

Examples of the anti-bacterial metal ions to substitute for these ions include silver, copper, zinc, mercury, tin, lead, bismuth, cadmium, chromium, and thallium ions; preferably silver, copper, or zinc ions, and more preferably silver ions.

The content of the anti-bacterial ions is preferably 0.1-15 mass % of the zeolite.

For example, anti-bacterial zeolite containing 0.1-15% of silver ion and 0.1-8 mass % of copper ion or zinc ion is preferable. On the other hand, zeolite can contain up to 20 mass % of ammonium ions; however, for the purpose of effectively preventing discoloration of the zeolite, 0.5-5% is preferable and 0. 5-3 mass % is more preferable. "Mass %" means the mass percentage in 110°C dry standard zeolite.

In the present invention, commercial products can be used for the anti-bacterial zeolite; the anti-bacterial zeolite can also be prepared by the following method, for example.

That is, zeolite is exposed to a mixed solution containing anti-bacterial metal ions such as silver ions, copper ions, and zinc ions, prepared in advance, to substitute the aforementioned ions for the exchangeable ions in the zeolite.

The exposure can be achieved by the batch method or continuous method (column method, for example) for 3-24 hours, preferably 10-24 hours, at 10-70°C, preferably 40-60°C.

The pH of the aforementioned mixed solution should be adjusted to 3-10, preferably 5-7. This adjustment is preferable because precipitation of silver oxide and such on the surface or in the pores of the zeolite can be prevented by this. Each ion in the mixed aqueous solution is usually supplied in the form of a salt. For example, silver ions are from silver nitrate, silver sulfate, silver perchlorate, diamminesilver nitrate, diamminesilver sulfate, etc copper ions are from copper nitrate (II), copper perchlorate, copper acetate, potassium tetracyanocuprate, copper sulfate, etc.; zinc ions are from zinc nitrate (II), zinc sulfate, zinc perchlorate, zinc thiocyanate, zinc acetate, etc. mercury ions are from mercury perchlorate, mercury nitrate, and mercury acetate; tin ions are from tin sulfate and such; lead ions are from lead sulfate, lead nitrate, etc.; bismuth ions are from bismuth chloride, bismuth iodide, etc cadmium ions are from cadmium perchlorate, cadmium sulfate, cadmium nitrate, and cadmium acetate ; chromium ions are from chromium perchlorate, chromium sulfate, chromium ammonium sulfate, chromium nitrate, etc.; thallium ions are from thallium perchlorate, thallium sulfate, thallium nitrate, thallium acetate, etc.

The anti-bacterial metal ion content in the zeolite can be controlled by adjusting the concentration of each ion (salt) in said mixed aqueous solution.

For example, in the case of anti-bacterial zeolite containing silver ions, an anti-bacterial zeolite with a silver ion content of 0.1-5% can be obtained by adjusting the silver ion concentration in said mixed aqueous solution to 0.002M/1-0.15M/1.

In the case of anti-bacterial zeolite additionally containing copper ions and zinc ions, an anti-bacterial zeolite with a copper ion content of 0.1-8% and a zinc ion content of 0.1-8% can be obtained by adjusting the silver ion concentration to 0.1M/1-0.85M/1 and the zinc ion concentration to 0.15M/1-1.2M/1 in said mixed aqueous solution.

For ion exchange of anti-bacterial zeolite, it is also possible to use solutions, each of which contains each ion, and expose the zeolite with these solutions one after another. The concentration of each ion in each aqueous solution can be determined based on the concentration of each ion in said mixed aqueous solution.

After the completion of the ion exchange, the zeolite is thoroughly rinsed and then dried. The drying is preferably done at 105°C-115°C, or under a reduced pressure (1-30 Torr) at 70-90°C.

Ion exchange for organic ions and/or for ions for which there isn't an adequate water soluble salt, such as tin and bismuth, can be done by using an organic solvent solution such as an alcohol or acetone to prevent precipitation of slightly soluble basic salts.

The blend ratio of the anti-bacterial zeolite in the skin treatment composition is not limited in particular. It is determined based on the reason why the anti-bacterial zeolite is added and also on the product form of the skin treatment composition. For example, when blended in as a preservative, the blend ratio is usually 0. 05-10 mass % of the total amount of the skin treatment composition.

As another example, when blended in as a bactericide, the blend ratio is usually 0.1-90 mass % of the total amount of the skin treatment composition, depending on the product form.

For example, for lotion or cream type skin treatment compositions 0.1-20 mass % of the total amount of the skin treatment composition is preferable; for powder type skin treatment compositions 0. 5-80 mass % of the total amount of the skin treatment composition is preferable; for stick type skin treatment compositions 0. 5-60 mass % of the total amount of the skin treatment composition is preferable; and for spray type skin treatment compositions 0. 5-50 mass % of the total amount of the skin treatment composition is preferable.

Polyoxyethylene polyoxypropylene 2-decyltetradecyl ether used in the present invention is a prior art ingredient of skin treatment compositions as a surfactant. It is usually blended into lotion as a solubilizing agent.

In the present invention, polyoxyethylene polyoxypropylene 2-decyltetradecyl ether (20-28 E.O.) (10-16 P.O.) is preferable; it specifically acts as an anti-staining agent for skin treatment compositions containing anti-bacterial zeolite.

Other surfactants known as solubilizing agents for perfume, such as polyoxyethylene (E.0. 60) hydrogenated castor oil, do not have an anti-staining effect for skin treatment compositions.

The blend ratio of the polyoxyethylene polyoxypropylene 2-decyltetradecyl ether (20-28 E.0.) (10-16 P.0.) is determined based on the blend ratio of the anti-bacterial zeolite and the product form. It is usually 0. 01-5 mass % of the total amount of the skin treatment composition.

When using the skin treatment composition of the present invention for applications such as odor eliminating agents and antiperspirant cosmetics, it is preferable to blend in an aluminum compound that is an antiperspirant.

In addition to the aforementioned essential ingredients, other ingredients commonly used in skin treatment compositions, for example one, two or more of those listed below, are blended as necessary in the skin treatment composition of the present invention; the preparation can be conducted for the target formulation with a conventional method.

### [Ingredients that can be blended in the present invention]

Examples of the powder ingredients include inorganic powders (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, barium sulfate, firing calcium sulfate (calcined gypsum), calcium phosphate, fluorine-apatite, hydroxy apatite, ceramic powder, metallic soaps (for example, zinc myristate, calcium palmitate, and aluminum stearate), and boron nitride); organic powders (for example, polyamide resin powder (nylon powder), polyethylene powder, poly methyl methacrylate powder, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ -iron oxide); inorganic yellow pigments (for example, yellow iron oxide and loess); inorganic black pigments (for example, black iron oxide and low oxides of titanium): inorganic purple pigments (for example, manganese violet, cobalt violet); inorganic green pigments (for example, chromium oxide, chromium hydroxide, and cobalt titanate); inorganic blue pigments (for example, ultramarine blue and Berlin blue); pearl pigment (for example, titania coated mica, titania coated bismuth oxychloride, titania coated talc, coloration titania coated mica, bismuth oxychloride, fish scale flakes); metal powder pigments (for example, aluminum powder, copper powder); organic pigments such as Zr, barium or aluminum rake (for example, organic pigments such as red 201, red 202, red 204, red 205, red 220, red 226, red 228, red 405, orange 203, orange 204, yellow 205, yellow 401 and blue 404, as well as red 3, red 104, red 106, red 227, red 230, red 401, red 505, orange 205, yellow 4, yellow 5, yellow 202, yellow 203, green 3 and blue 1; and natural colors (for example, chlorophyll and β -carotene).

Examples of the liquid fats and oils include avocado oil, tsubaki oil, turtle fatty acid, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japan gimlet oil, jojoba oil, germ oil, and triglycerin.

Examples of the solid fats and oils include cacao butter, coconut oil, hydrogenated coconut oil, palm oil, palm kernel oil, Japanese core wax nucleus oil, hydrogenated oil, Japanese core wax, and hydrogenated castor oil.

Examples of the waxes include beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, P0E lanolin alcohol acetate, POE cholesterol ether, lanolin fatty acid polyethylene glycol, POE hydrogenated lanolin ethyl alcohol ether, ceresin, and microcrystalline wax.

Examples of the hydrocarbon oils include liquid petrolatum, ozocerite, squalane, pristane, paraffin, squalene, and petrolatum.

Examples of the higher fatty acids include lauric acid, myristic acid, palmitic acid, stearic acid, behenic acid, oleic acid, undecylenic acid, isostearic acid, linolic acid, linoleic acid, eicosapentaenoic acid (EPA), and docosahexaenoic acid (DHA).

Examples of the higher alcohols include straight chain alcohols (for example, lauryl alcohol, cetyl alcohol, stearyl alcohol, behenyl alcohol, myristyl alcohol, oleyl alcohol, and cetostearyl alcohol) and branched chain ethyl alcohols (for example, mono stearyl glycerin ether (batyl alcohol), 2-decyltetradecynol, lanolin alcohol, cholesterol, phytosterol, hexyl dodecanol, iso stearyl alcohol, and octyl dodecanol).

Examples of the ester oils include isopropyl myristate, cetyl octanoate, octyl dodecyl myristate, isopropyl palmitate, butyl stearate, hexyl laurate, myristil myristate, decyl oleate, dimethyl hexyl decyl octanoate, cetyl lactate, myristil lactate, lanolin acetate, iso cetyl stearate, iso cetyl isostearate, cholesteryl hydroxy 12-stearate, di-2-ethylene glycol ethylhexanoate, dipentaerythritol fatty acid ester, n-alkylene glycol monoisostearate, neopentyl glycol dicaprate, diisostearyl malate, glyceryl di-2-heptylundecanoate, trimethylolpropane tri-2-ethylhexanoate, trimethylolpropane triisostearate, tetra-2-pentaerythritol ethylhexanoate, glycerin tri-2-ethylhexanoate, glyceryl trioctanoate, glycerin triisopalmitate, trimethylolpropane triisostearate, cetyl 2-ethyl hexanoate, 2-ethylhexyl palmitate, glycerin trimyristate, tri-2-heptyl undecanoic acid glyceride, methyl castor oil fatty acid, oleyl oleate, aceto glyceride, 2-heptyl undecyl palmitate, diisobutyl adipate, 2-octyldodecyl N-lauroyl-L-glutamate, di-2-heptyl undecyl adipate, ethyl laurate, di-2-ethylhexyl sebacate, 2-hexyl decyl myristate, 2-hexyl decyl palmitate, 2-hexyl decyl adipate, diisopropyl sebacate, 2-ethylhexyl succinate, and triethyl citrate.

Examples of the silicone oils include chain polysiloxanes (for example, dimethylpolysiloxane, methylphenyl polysiloxane, and diphenyl polysiloxane); ring polysiloxanes (for example, octamethylcyclotetrasiloxane, decamethyl cyclopenta siloxane, and dodecamethyl cyclohexa siloxane), silicone resins forming a three-dimensional network structure, silicone rubbers, and various modified polysiloxanes (amino-modified polysiloxane, polyether-modified polysiloxane, alkyl-modified polysiloxane, and fluorine-modified polysiloxane).

Examples of the anionic surfactants include fatty acid soaps (for example, sodium laurate and sodium palmitate, higher alkyl sulfuric ester salts (for example, sodium lauryl sulfate and potassium laurylsulfate) ; alkylether sulfuric ester salts (for example, POE-triethanolamine laurylsulfate and sodium POE-lauryl sulfate); N-acyl sarcosinic acids (for example, sodium N-lauroyl sarcosinate); higher fatty acid ester sulfates (for example, hydrogenated coconut oil aliphatic acid glycerin sodium sulfate); N-acyl glutamates (for example, mono sodium N-lauroylglutamate, disodium N-stearoylglutamate, and sodium N-myristoyl-L-glutamate); sulfated oils (for example, turkey red oil); POE-alkylether carboxylic acid; POE-alkylarylether carboxylate; α -olefin sulfonate; higher fatty acid ester sulfonates; sec-alcohol sulfates; higher fatty acid alkyl amide sulfates; sodium lauroyl monoethanolamine succinates; ditriethanolamine N-palmitoylaspartate; and sodium caseinate.

Examples of the cationic surfactants include alkyltrimethylammonium salts (for example, stearyltrimethyl ammonium chloride and lauryltrimethyl ammonium chloride) alkylpyridinium salts (for example, cetylpyridinium chloride), distearyldimethylammonium chloride dialkyldimethylammonium salt; poly (N,N'-dimethyl-3,5-methylene piperidinium) chloride; alkyl quaternary ammonium salts; alkyl dimethylbenzyl ammonium salts; alkyl isoquinolinium salts; dialkylmorpholine salts; POE alkyl amines; alkyl amine salts; polyamine fatty acid derivatives; amylalcohol fatty acid derivatives; benzalkonium chloride; and benzethonium chloride.

Examples of the ampholytic surfactants include: imidazoline type ampholytic surfactants (for example, 2-undecyl-N, N, N-(hydroxyethyl carboxymethyl)-2-imidazoline sodium salt and 2-coco yl-2-imidazolinium hydroxide-1-carboxyethyloxy 2 sodium salt); and betaine type surfactants (for example,
2-heptadecyl-n-carboxymethyl-n-hydroxyethyl imidazolinium betaine, lauryldimethylaminoacetic acid betaine, alkyl betaine, amide betaine, and sulfobetaine).

Examples of the lipophilic nonionic surfactant include sorbitan fatty acid esters (for example, sorbitan mono oleate, sorbitan mono isostearate, sorbitan mono laurate, sorbitan mono palmitate, sorbitan mono stearate, sorbitan sesqui oleate, sorbitan trioleate, diglycerol sorbitan penta-2-ethylhexylate, and diglycerol sorbitan tetra-2-ethylhexylate); glycerin polyglycerin aliphatic acids (for example, mono-cottonseed oil fatty acid glycerin, glyceryl monoerucate, glycerin sesquioleate, glyceryl monostearate, α , α '-glyceryl oleate pyroglutamate, and glyceryl mono stearate mono malate); propylene glycol fatty acid esters (for example, propylene glycol monostearate); hydrogenated castor oil derivatives; and glycerin alkylethers.

Examples of the hydrophilic nonionic surfactant include: POE-sorbitan fatty acid esters (for example, POE-sorbitan monooleate, POE-sorbitan monostearate, POE-sorbitan monoolate, and POE-sorbitan tetraoleate); POE sorbitol fatty acid esters (for example, POE sorbitol monolaurate, POE-sorbitol monooleate, POE-sorbitolpentaoleate, and POE-sorbitol monostearate); POE-glycerin fatty acid esters (for example, POE-monooleates such as POE-glycerin monostearate, POE-glycerin monoisostearate, and POE-glycerin triisostearate); POE-fatty acid esters (for example, POE-distearate, POE-monodioleate, and ethylene glycol distearate); POE-alkylethers (for example, POE-lauryl ether, POE-oleyl ether, POE-stearyl ether, POE-behenyl ether, POE-2-octyl dodecyl ether, and POE-cholestanol ether); POE/POP-alkylethers (for example, POE/POP-cetyl ether, POE/POP-2-decyl tetradecyl ether, POE/POP-monobutyl ether, POE/POP-lanolin hydrate, and POE/POP-glycerin ether); POE-castor oil hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamic monoisostearic diester, and POE-hydrogenated castor oil maleic acid); POE-beeswax/lanolin derivatives (for example, POE-sorbitol beeswax) ; alkanol amides (for example, coconut fatty acid diethanol amide, lauric acid monoethanol amide, and aliphatic acid isopropanol amide); POE-propylene glycol fatty acid esters; POE-alkyl amines; POE-fatty acid amides; sucrose fatty acid esters; alkyl ethoxy dimethylamine oxides; and trioleyl phosphoric acid.

Examples of the humectant include polyethylene glycol, propylene glycol, glycerin, 1,3-butylene glycol, xylitol, sorbitol, maltitol, chondroitin sulfate, hyaluronic acid, mucoitin sulfuric acid, charonic acid, atelocollagen, cholesteryl-12-hydroxy stearate, sodium lactate, bile salt, dl-pyrrolidone carboxylic acid salt, short chain soluble collagen, diglycerin (E0)P0 adduct, chestnut rose fruit extract, yarrow extract, and sweet clover extract.

Examples of the natural water-soluble polymer include: plant-type polymers {for example, gum arabic, gum tragacanth, galactan, guar gum, carob gum, karaya gum, carrageenan, pectin, agar, quince seed (Cydonia oblonga), algae colloids (brown algae extract), starches (rice, corn, potato, and wheat), and glycyrrhizic acid}; microorganism-type polymers (for example, xanthan gum, dextran, succinoglucan, and pullulan); and others (for example, fish-derived collagen, fish-derived gelatin, wheat protein, and silk proten).

Examples of the semisynthetic water-soluble polymers include: starch-type polymers (for example, carboxymethyl starch and methylhydroxypropyl starch); cellulosic polymers (for example, methyl cellulose, ethyl cellulose, methylhydroxypropyl cellulose, hydroxyethyl cellulose, cellulose sodium sulfate, hydroxypropyl cellulose, carboxymetyl-cellulose, sodium carboxymethyl cellulose, crystal cellulose, and cellulose powder); and alginic acid-type polymers (for example, sodium alginate and propyleneglycol alginate).

Examples of the synthetic water-soluble polymers include: vinyl polymers (for example, polyvinyl alcohol, polyvinyl methyl ether, polyvinylpyrrolidone, and carboxy vinyl polymer); polyoxyethylene-type polymers (for example, a copolymer of polyethylene glycol 20, 000, 40,000, or 60,000 and polyoxyethylene polyoxypropylene); acrylic polymers (for example, sodium polyacrylate, polyethylacrylate, and polyacrylamide); polyethyleneimine; and cationic polymers.

Examples of the thickeners include: gum arabic, carrageenan, karaya gum, gum tragacanth, carob gum, quince seed (Cydonia oblonga), casein, dextrin, gelatin, sodium pectate, sodium arginate, methyl cellulose, ethyl cellulose, CMC, hydroxy ethyl cellulose, hydroxypropyl cellulose, PVA, PVM, PVP, sodium polyacrylate, carboxy vinyl polymer, locust bean gum, guar gum, tamarind gum, cellulose dialkyl dimethylammonium sulfate, xanthan gum, aluminum magnesium silicate, bentonite, hectorite, A1Mg silicate (beagum), laponite, and silicic acid anhydride.

Examples of the ultraviolet absorbents include the following compounds.

### (1) Benzoic acid-type ultraviolet absorbents

For example, p-aminobenzoic acid (hereafter abbreviated as PABA), PABA monoglycerin ester, N, N-dipropoxy PABA ethyl ester, N, N-diethoxy PABA ethyl ester, N,N-dimethyl PABA ethyl ester, N, N-dimethyl PABA butyl ester, and N,N-dimethyl PABA ethyl ester.

### (2) Anthranilic acid-type ultraviolet absorbents

For example, homo mentyl-N-acetyl anthranilate.

### (3) Salicylic acid-type ultraviolet absorbents

For example, amyl salicylate, mentyl salicylate, homo mentyl salicylate, octyl salicylate, phenyl salicylate, benzyl salicylate, and p-isopropanol phenyl salicylate.

### (4) Cinnamic acid-type ultraviolet absorbents

For example, octyl cinnamate, ethyl-4-isopropyl cinnamate, methyl-2 ,5-diisopropyl cinnamate, ethyl-2 ,4-diisopropyl cinnamate, methyl-2 ,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-*β* -phenyl cinnamate, 2-ethylhexyl-α -cyano- β -phenyl cinnamate, and glyceryl mono-2-ethyl hexanoyl-di-p-methoxy cinnamate.

### (5) Triazine-type ultraviolet absorbents

For example, bisresorsinyl triazine.

More specifically,
bis{[4-(2-ethylhexyloxy)-2-hydroxylphenyl)-6-(4-me thoxyphenyl)1,3,5-triazine,
2, 4, 6-tris {4-(20ethylhexyloxycarbonyl) anilino} 1, 3, 5-triazine, etc.

### (6) Other ultraviolet absorbents

For example, 3-(4' -methylbenzylidene) - d,1-camphor, 3-benzylidene-d,1-camphor, 2-phenyl-5-methyl benzoxazol, 2-(2 '-hydroxy-5'-methylphenyl) benzotriazol, 2-(2'-hydroxy-5'-t-octylphenyl) benzotriazol, 2-(2'-hydroxy-5'-methylphenyl benzotriazol, dibenzaladine, dianisoylmethane, and 4-methoxy-4'-t-butyl dibenzoyl-methane, 5-(3,3-dimethyl-2-norbornylidene)-3-pentane-2-one. Pyridazinone derivatives such as dimorpholino pyridazine.

Examples of the sequestering agents include: 1-hydroxy ethane-1,1-diphosphonic acid, 1-hydroxy ethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, and succinic acid.

Examples of the lower alcohols include ethanol, propanol, isopropanol, isobutanol, and t-butyl alcohol.

Examples of the polyhydric alcohols include: dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritol such as 1, 2, 6-hexanetriol); pentahydric alcohols (for example, xylitol); hexahydric alcohols (for example, sorbitol, mannitol); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkylethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methyl hexyl ether, ethylene glycol isoamyl ether, ethylene glycol benzyl ether, ethylene glycol isopropyl ether, ethylene glycol dimethylether, ethylene glycol diethyl ether, and ethylene glycol dibutyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycolmonomethyl ether acetate, propylene glycol monoethyl ether acetate, propylene glycol monopropyl ether acetate, and propylene glycol monophenyl ether acetate); glycerin mono alkyl ethers (for example, xylyl alcohol, selachyl alcohol, and batyl alcohol); sugar alcohols (for example, sorbitol, maltitol, maltotriose, mannitol, sucrose, erythritol, glucose, fructose, starch amylolysis sugar, maltose, xylitose, and alcohol prepared by the reduction of starch amylolysis sugar); glysolid; tetrahydro furfuryl alcohol; POE-tetrahydro furfuryl alcohol; POP-butyl ether; POP.POE-butyl ether; tripolyoxypropylene glycerin ether; POP-glycerin ether, POP-glycerin ether phosphoric acid; POP/POE-pentane erythritol ether, and polyglycerin.

Examples of the monosaccharides include: trioses (for example, D-glyceryl aldehyde and dihydroxyacetone); tetroses (for example, D-etythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose) ; heptoses (for example, aldoheptose and heprose) ; octoses (for example, octurose) ; deoxysugars (for example, 2-deoxy-D-ribose, 6-deoxy-L-galactose, and 6-deoxy-L-mannose); amino sugars (for example, D-glucosamine, D-galactosamine, sialic acid, amino uronic acid, and muramic acid) ; and uronic acid (for example, D-glucuronic acid, D-mannuronic acid, L-guluronic acid, D-galacturonic acid, and L-iduronic acid).

Examples of the oligosaccharides include sucrose, umbelliferose, lactose, planteose, isolignoses, α, α -trehalose, raffinose, lignoses, umbilicine, stachyose and verbascose.

Examples of the polysaccharides include cellulose, quince seed, chondroitin sulfate, starch, galactan, dermatan sulfate, glycogen, gum arabic, heparan sulfate, hyaluronic acid, traganth gum, keratan sulfate, chondroitin, xanthan gum, mucoitin sulfuric acid, guar gum, dextran, kerato sulfate, locustbean gum, succinoglucane, and charonic acid.

Examples of the amino acids include neutral amino acids (for example, threonine and cysteine) and basic amino acids (for example, hydroxylysine). Examples of the amino acid derivatives include sodium acyl sarcosinate (sodium N-lauroyl sarcosinate), acyl glutamate, acyl *β* -alanine sodium, glutathione, and pyrrolidone carboxylic acid.

Examples of the organic amines include monoethanolamine, diethanolamine, triethanolamine, morpholine, triisopropanolamine, 2-amino-2-carbinyl-1,3-propanediol, and 2-amino-2-carbinyl-1-propanol.

Examples of the high polymer emulsions include acrylic resin emulsions, ethyl polyacrylate emulsions, acryl resin liquids, polyacrylic alkyl ester emulsions, polyvinyl acetate resin emulsions, and natural rubber latex.

Examples of the pH adjustment agents include buffers such as lactic acid-sodium lactate, citric acid-sodium citrate, and succinic acid-sodium succinate.

Examples of the vitamins include vitamins A, B1, B2, B6, C and E as well as their derivatives, pantothenic acid and its derivatives, and biotin.

Examples of the antioxidants include tocopherols, dibutyl hydroxytoluene, butyl hydroxyanisole, and gallic ester.

Examples of the antioxidation auxiliary agents include phosphoric acid, citric acid, ascorbic acid, maleic acid, malonic acid, succinic acid, fumaric acid, cephalin, hexameta phosphate, phytic acid, and ethylene diamine tetraacetic acid.

Examples of other possible ingredients include antiseptics (methylparaben, ethylparaben, butylparaben, and phenoxyethanol); anti-inflammatory agents (for example, glycyrrhizic acid derivatives, glycyrrhetinic acid derivatives, salicylic acid derivatives, hinokitiol, zinc oxide, and allantoin); whitening agents (for example, creeping saxifrage extract, arbutin, tranexamic acid, L-ascorbic acid, magnesium L-ascorbyl phosphate, L-ascorbic acid glucosie, and potassium 4-methoxysalicylate); various extracts (for example, Phellodendri Cortex, goldthread, lithospermum root , Paeonia lactiflora, Swertia japonica, Birch, sage, loquat, carrot, aloe, Malva sylvestris, Iris, grape, Coix ma-yuen, sponge gourd, lily, saffron, Cnidium officinale, sheng jiang, Hypericum erectum, Ononis, garlic, Guinea pepper, chen pi, Ligusticum acutilobum, and seaweed), activators (royal jelly, photosensitizer, and cholesterol derivatives); blood circulation promoting agents (for example, nonyl acid valenyl amide, nicotinic acid benzyl esters, nicotinic acid *β*-butoxy ethyl esters, capsaicin, gingeron, cantharis tincture, Ichthammol, tannic acid, α -borneol, tocopherol nicotinate, inositol hexanicotinate, cyclandelate, cinnarizine, tolazoline, acetylcholine, verapamil, cepharanthine, and *β* -orizanol); ; anti-seborrhea agents (for example, sulfur and thiantol); and antiinflammatory agents (for example, thiotaurine and hypotaurine); and bactericides (for example, benzoic acid and its salts, isopropylmethyl phenol, undecylenic acid and its salts, undecylenic acid monoethanol amide, cetyltrimethyl ammonium chloride, cetylpyridinium chloride, benzalkonium chloride, benzethonium chloride, alkyldiaminoethylglycine chloride, chlorhexidine chloride, orthophenyl phenol, chlorhexidine gluconate, cresol, chloramine T, chlorxylenol, chlorcresol, chlorfenesin, chlorobutanol, 5-chloro-2-methyl-4-isothiazoline-3-one, salicylic acid and its salts, 1,3-dimethylol-5,5-dimethylhidantoin, alkylisoquinolium bromide, domiphen bromide and its salt, sorbic acid and its salts, thymol, thylum, thiram, dehydroacetic acid and its salt, triclosan, trichlorocarbanilide, p-oxybenzoic ester, p-chlorphenol, halocarban, pyrogallol, phenol, hexachlorophene, 2-methyl-4-isothiazoline-3-one, NN"-Methylenebis(N'-(3-hydroxymethyl-2, 5-dioxo-4-i midazolidinyl)urea), sodium layroylsarcosine, and resorcin).

### EXAMPLES

The present invention is described in detail below by referring to Examples. The present invention is not limited to these examples. The blend ratios are in mass-percentage units unless specified otherwise.

### [Invention of claim 1]

Recipes shown in Table 1-1 and Table 1-2 were used to prepare powder lotion-type antiperspirant lotions and the degree of discoloration was evaluated visually. For Comparative examples, a recipe containing no trisodium ethylenediaminehydroxyethyl triacetate and a recipe containing a chelating agent EDTA-3Na^{.}2H₂O instead of trisodium ethylenediaminehydroxyethyl triacetate were investigated. The degree of discoloration was evaluated by giving O to those that are within the acceptable range for skin treatment composition, and X to those that are outside of this range.

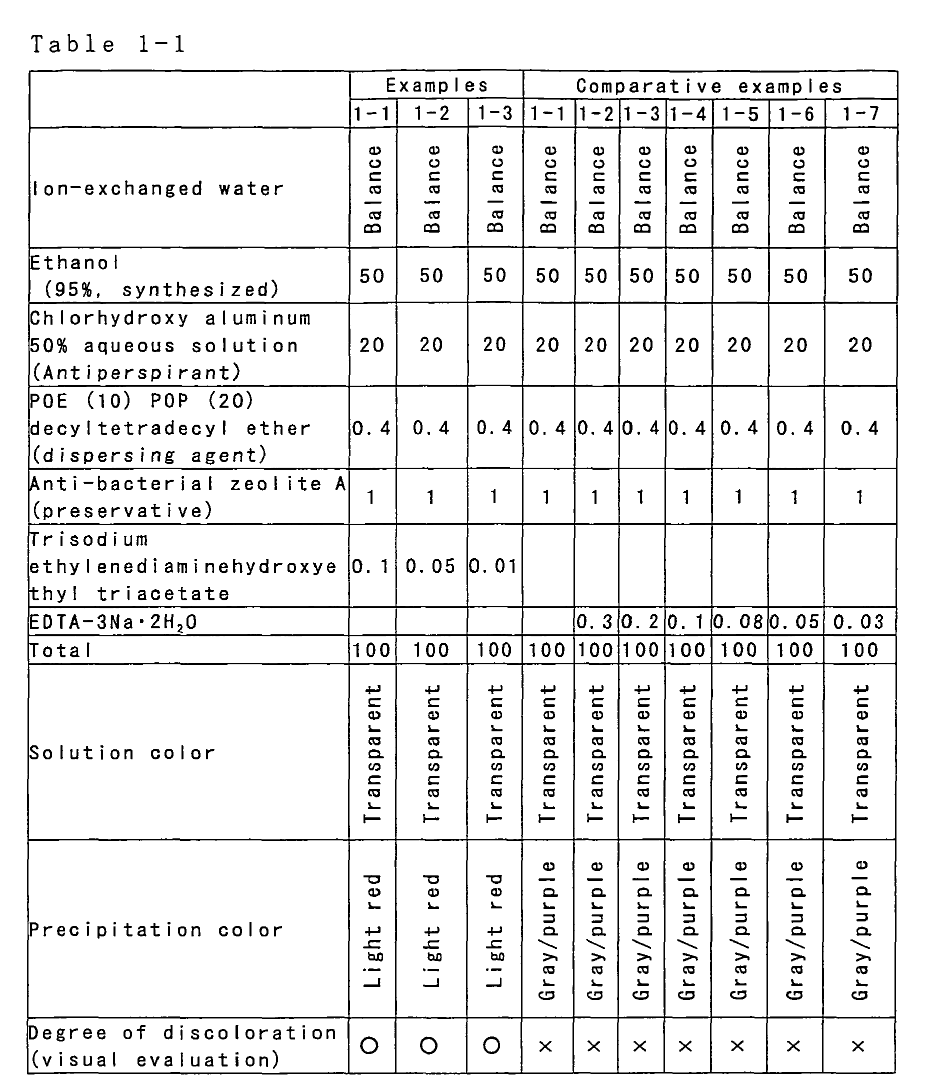

Anti-bacterial zeolite A: Zeolite containing silver ions and zinc ions (average particle size approximately 1.5 micrometers)

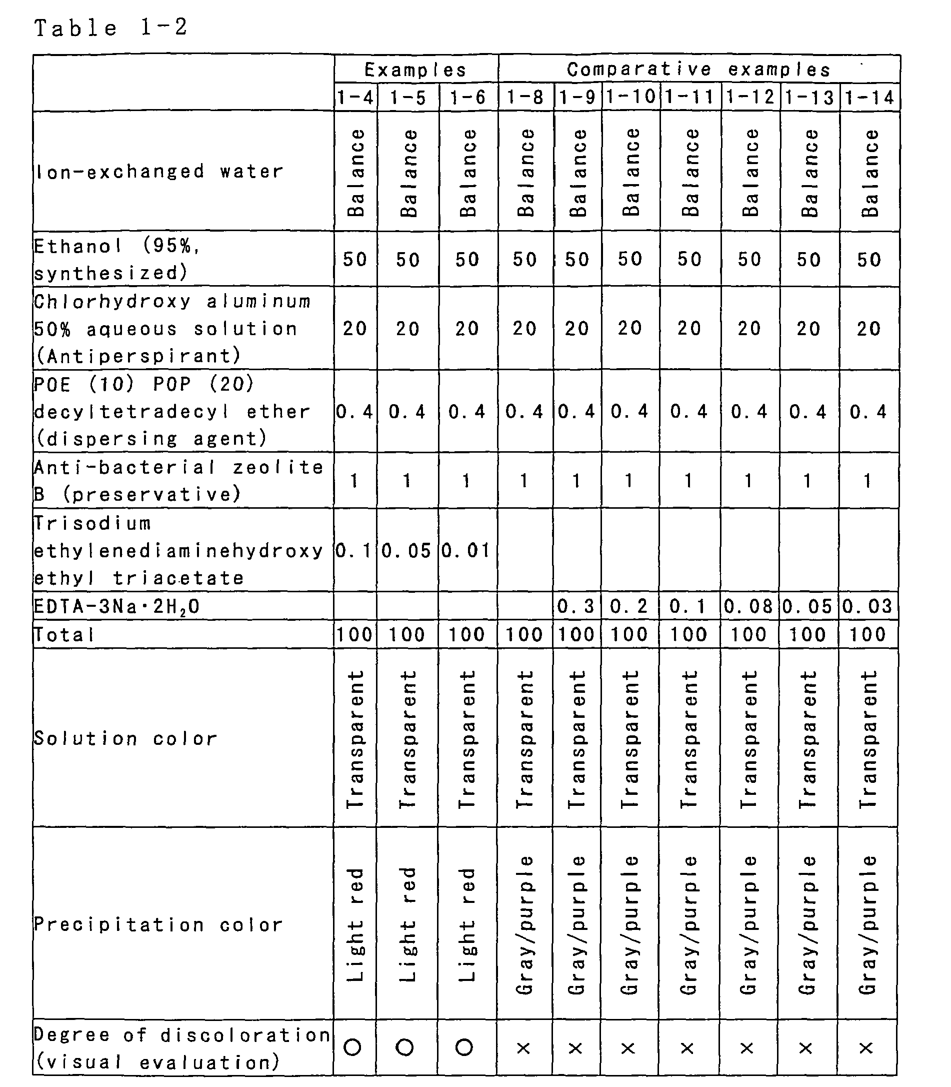

Anti-bacterial zeolite B: Zeolite containing silver ions, zinc ions, and ammonium ions (Zeomic AJ10N from Sinanen Zeomic Co., Ltd., average particle size approximately 1.5 micrometers)

The aforementioned results show that Comparative examples that do not contain trisodium ethylenediaminehydroxyethyl triacetate and Comparative examples that contain a chelating agent EDTA-3Na·2H₂O exhibit discoloration of precipitated white powder of anti-bacterial silver zeolite into gray/purple, resulting in a larger degree of discoloration.

On the other hand, Examples containing trisodium ethylenediaminehydroxyethyl triacetate exhibit only slight red discoloration of the precipitated anti-bacterial silver zeolite; and the degree of discoloration is very small and within the allowable range for skin treatment compositions; which indicates a superior antidiscoloration effect.

Other Examples of the present invention are shown below.

### Example 1-7: Pressed powder

| | |
|---|---|
| Chlorhydroxy aluminum | 5 mass % |
| Zinc oxide (zinc flower) | 5 |
| Talc | 76.99 |
| Liquid petrolatum | 3 |
| Anti-bacterial zeolite B | 10 |
| Trisodium ethylenediaminehydroxyethyl triacetate | 0.01 |
| Example 1-8: Loose powder | |
| Chlorhydroxy aluminum | 5 mass % |
| Zinc oxide (zinc flower) | 5 |
| Talc | 79.99 |
| Anti-bacterial zeolite B | 10 |
| Trisodium ethylenediaminehydroxyethyl | triacetate |
| | 0.01 |

### Example 1-9: Lotion-type spray

| | |
|---|---|
| (Stock solution recipe) | |
| Purified water | 10 mass % |
| Chlorhydroxy aluminum | 10 |
| Anhydrous ethyl alcohol | 73.9 |
| Isopropyl myristate | 2 |
| 1,3-butylene glycol | 3 |
| Anti-bacterial zeolite B | 1 |
| Trisodium ethylenediaminehydroxyethyl triacetate | 0.1 |
| (Filler recipe) | |
| Stock solution | 50 |
| LPG | 50 |

### Example 1-10: Powder spray

| | |
|---|---|
| Chlorhydroxy aluminum | 20 mass % |
| Silicic acid anhydride | 15 |
| Talc | 20.21 |
| Zinc oxide (zinc flower) | 5 |
| Isopropyl myristate | 21.79 |
| Dimethyl polysiloxane | 10 |
| Sorbitan fatty acid ester | 3 |
| Anti-bacterial zeolite B | 5 |
| Trisodium ethylenediaminehydroxyethyl triacetate | 0.1 |
| (Filler recipe) | |
| Stock solution | 10 |
| LPG | 90 |

### Example 1-11: Powder spray

| | |
|---|---|
| Chlorhydroxy aluminum | 20 mass % |
| Silicic acid anhydride | 15 |
| Talc | 20.21 |
| Zinc oxide (zinc flower) | 5 |
| Isopropyl myristate | 21.79 |
| Polyoxyethylene/polyoxypropylene random polymermethyl ether | 10 |
| Sorbitan fatty acid ester | 3 |
| Anti-bacterial zeolite B | 5 |
| Trisodium ethylenediaminehydroxyethyl triacetate (Filler recipe) | 0. 1 |
| Stock solution | 10 |
| LPG | 90 |

### Example 1-12: Stick

| | |
|---|---|
| Chlorhydroxy aluminum | 20 mass % |
| Talc | 7. 9 |
| Zinc oxide (zinc flower) | 5 |
| Solid petrolatum wax | 2 |
| Stearyl alcohol | 8 |
| Liquid petrolatum | 15 |
| Cyclic dimethyl polysiloxane | 36 |
| Sorbitan fatty acid ester | 1 |
| Anti-bacterial zeolite B | 5 |
| Trisodium ethylenediaminehydroxyethyl triacetate | 0.1 |

### Example 1-13: Cream

| | |
|---|---|
| Purified water | 45 mass % |
| Squalane | 20 |
| Cyclic dimethyl polysiloxane | 15 |
| Glyceryl diisostearate | 3 |
| Diethoxyethyl succinate | 5 |
| Organically modified montmorillonite | 1.5 |
| 1,3-butylene glycol | 5.49 |
| Anti-bacterial zeolite A | 5 |
| Trisodium ethylenediaminehydroxyethyl triacetate | 0.01 |

### Example 1-14: Emulsion

| | |
|---|---|
| Purified water | 20 mass % |
| Chlorhydroxy aluminum | 20 |
| Octyl-p-methoxycinnamate | 5 |
| Oxybenzone | 3 |
| 4-tert butyl-4'-methoxybenzoylmethane | 1 |
| Hydrophobically treated zinc oxide | 5 |
| Polyoxyethylene/polypropylene random polymer methyl ether | 10 |
| Silicone oil | 15 |
| Silicone resin | 1 |
| Glyceryl diisostearate | 1 |
| Organically modified montmorillonite | 0.5 |
| 1,3-butylene glycol | 5.49 |
| Anti-bacterial zeolite B | 13 |
| Trisodium ethylenediaminehydroxyethyl triacetate | 0.01 |

### Example 1-15: Ointment

| | |
|---|---|
| Purified water | 53.74 mass % |
| Chlorhydroxy aluminum | 20 |
| Glycerin | 10 |
| 1,3-butylene glycol | 3 |
| Caustic potash | 0.25 |
| Stearic acid | 2 |
| Stearic acid monoglyceride | 2 |
| Cetanol | 1 |
| Liquid petrolatum | 5 |
| Petrolatum | 2 |
| Anti-bacterial zeolite B | 1 |
| Trisodium ethylenediaminehydroxyethyl triacetate | 0.01 |

### Example 1-16: Gel

| | |
|---|---|
| Purified water | 63.27 mass % |
| Chlorhydroxy aluminum | 20 |
| Dipropylene glycol | 5 |
| PEG 1500 | 5.5 |
| Carboxyvinyl polymer | 0.4 |
| Methylcellulose | 0.2 |
| POE (15) oleyl alcohol ether | 0.5 |
| Potassium hydroxide | 0.1 |
| EDTA | 0.02 |
| Anti-bacterial zeolite B | 5 |
| Trisodium ethylenediaminehydroxyethyl triacetate | 0.01 |
| Example 1-17: Wet sheet | |
| Purified water | 62.81 mass % |
| Anhydrous ethyl alcohol | 35 |
| Polyoxyethylene hydrogenated castor | oil |
| | 0.1 |
| Citric acid (food) | 0.02 |
| Sodium citrate | 0.06 |
| Anti-bacterial zeolite B | 2 |
| Trisodium ethylenediaminehydroxyethyl triacetate | 0.01 |

### [Invention of claims 2-6]

### Examples 2-1 to 2-3, Comparative examples 2-1 to 2-4

### [Deodorant powder spray]

Deodorant powder sprays having compositions of Examples 2-1 to 2-3 and Comparative examples 2-1 to 2-4, shown in Table 2-1, were prepared with the following preparation method, and the stability (anti-discoloration properties), usability, and the deodorizing effect of the formulations were evaluated with the method described below. The evaluation results are also shown in Table 2-1. Preparation method

The powder ingredients are thoroughly mixed with a Henschel mixer to prepare the powder portion. The oil ingredients, surfactant and such are mixed and dissolved with a blender to prepare the oil phase portion. An aluminum aerosol can with an inside volume of 80 mL is filled with 5.3 g of the powder portion and 2.9 g of the oil phase portion; after clinching, the propellant (LPG 0.18 MPa/20°C) is added to obtain a powder spray.

### (1) Usability

The following tests were conducted using the deodorant sprays that had been stored undisturbed at room temperature for 6 months. 40 test subjects sprayed Examples and Comparative examples on either their left or right armpit from 10 cm away for three seconds and then spread the sample with a hand to conduct a sensory evaluation of the tactile sensation during use. Graininess is believed to be caused by aggregation of the powder.

### <Evaluation criteria>

The number of test subjects who determined the tactile sensation during use was without graininess after the sensory evaluation was indicated.
A: 32 or more
B: 20 or more and 31 or less
C: 19 or less

### (2) Deodorizing effect (armpit odor)

In summer when perspiration tends to occur, 40 male panelists who were aware of their armpit odor were used in the following method and a judge conducted sensory evaluation. The test samples were randomly allotted (left and right were separate);one person who is not a panelist or a judge was in charge of sample allotment and maintenance of the allotment key codes for the purpose of the double-blind testing. Armpits of the panelists were wiped with 70% ethanol until they didn't smell, and the samples were used from 10 cm away for three seconds. Each panelist was prohibited from bathing, showering, or cleaning the armpits; after 24 hours the judge evaluated the degree of smell from the left and right armpits using the following criteria.

The evaluation was based on the six-point method based on the following criteria; the average of 40 male panelists was used for the evaluation results. A higher number indicates stronger smell.

### (Evaluation)

0 points: No smell
1 point: Very faint smell
2 points: Faint smell
3 points: Medium smell
4 points: Somewhat strong smell
5 points: Strong smell

### Evaluation results

A: 0 points or more and less than 2 points
B: 2 points or more and less than 3 points
C: 3 points or more

### (3) Anti-discoloration properties

The deodorant powder spray in an aerosol container was sprayed on white sheets of paper from approximately 10 cm away for three seconds to prepare samples; samples after being exposed to sunlight for three hours were compared with those with no sunlight exposure to ascertain whether the color of each sample changed or not; evaluation was conducted visually by specialized researchers. The evaluation criteria are as follows. Samples with less color changes are more preferable for commercial products and have more formulation stability.

### <Evaluation criteria>

A: No color change is detected.
B: Slight color change is detected.
C: Obvious color change is detected.

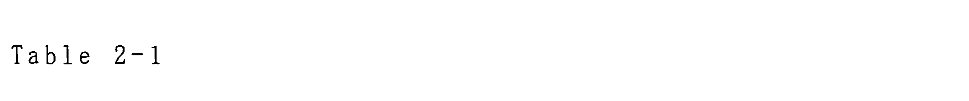

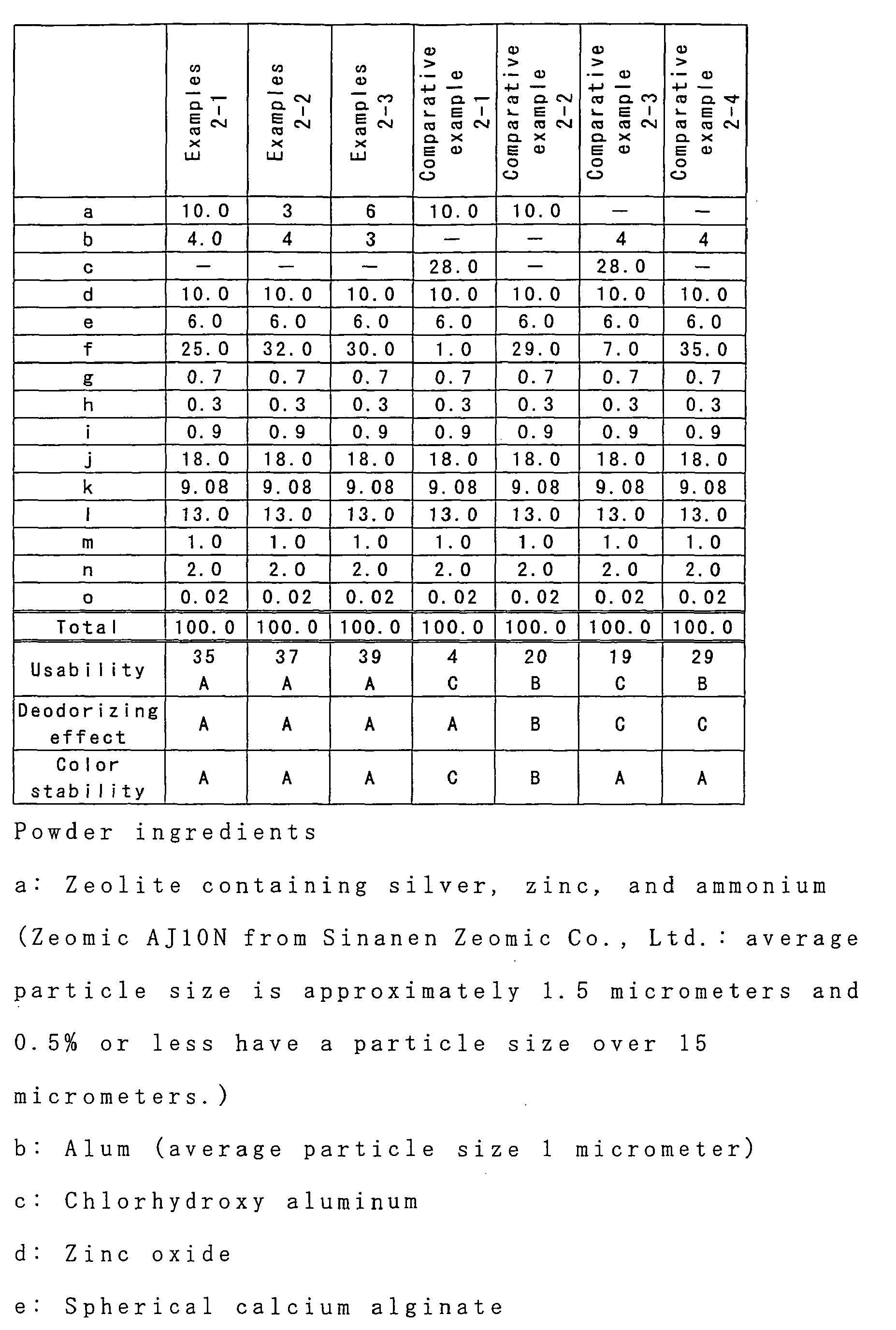

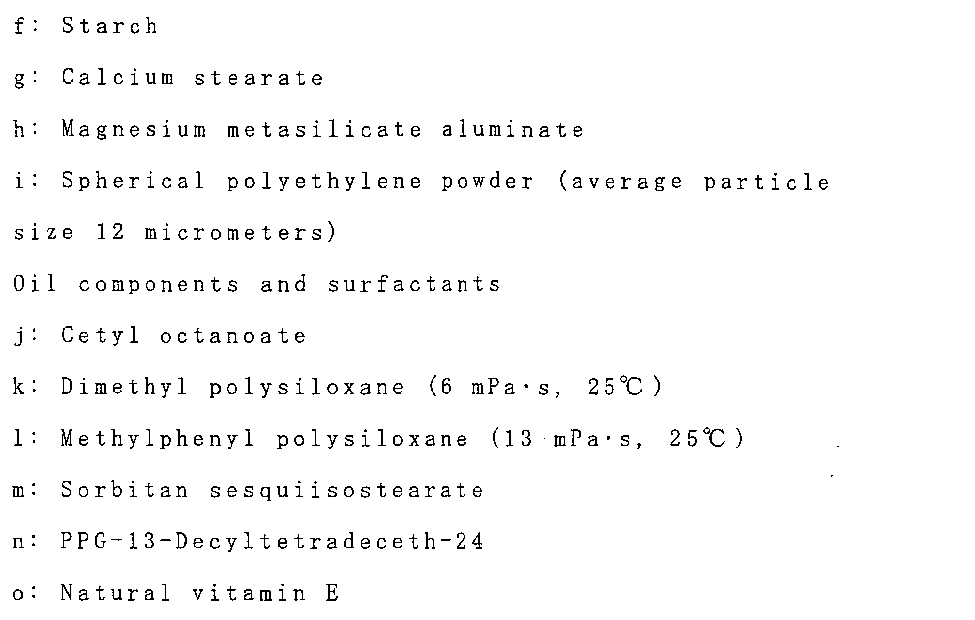

The aforementioned Examples and Comparative examples show that the deodorant powder sprays of the present invention are superior in terms of usability, deodorizing properties, and anti-discoloration properties compared with Comparative examples.

Also, the deodorant powder sprays of Examples did not exhibit powder aggregation and had superior dispersibility.

Other Examples of the skin treatment composition of the present invention are shown below.

### Example 2-4

### [Pressed powder type deodorant cosmetic]

| | |
|---|---|
| (Powder portion) | |
| Zeolite containing silver ions and ammonium ions | |
| (average particle size is approximately 3 | |
| micrometers; 0.5% or less have a particle size over | |
| 15 micrometers.) | 4.0 mass % |
| Alum (average particle size 0.05 micrometers) | 1. 5 |
| Chlorhydroxy aluminum | 0.5 |
| Zinc oxide | 3.0 |
| Talc | 87.0 |
| (Oil components) | |
| Methylphenyl polysiloxane (13 mPa·s, 25°C) | 3. 0 |
| Liquid petrolatum | 1.0 |
| (Additives) | |
| Perfume | Appropriate amount |

(Preparation method) The powder portion is mixed with a Henschel mixer; the oil components and the additive are added to this mixture, which is then crushed with a 5HP pulverizer (from Hosokawa Micron Ltd.) and molded in a medium plate to obtain a pressed powder type deodorizing cosmetic.

The obtained pressed powder type deodorizing cosmetic does not exhibit caking during use, and has good usability (no graininess) as well as sufficient deodorizing effects and anti-discoloration properties.

### Example 2-5

### [Deodorant powder]

| | |
|---|---|
| Dried alum (average particle size 4 micrometers) | 13.0 mass % |
| Zeolite containing silver ions and ammonium ions | |
| (average particle size is approximately 2 | |
| micrometers; 1% or less have a particle size over 15 | |
| micrometers.) | 7.0 |
| Spherical nylon powder | 5.0 |
| Dimethyl polysiloxane (molecular weight 450,000) | 1. 0 |
| Synthesized isoparaffin | 1.0 |
| Perfume | Appropriate amount |
| Talc | 73.0 |

(Preparation method) The aforementioned ingredients are mixed one after another with a Henschel mixer to obtain deodorant powder. The obtained deodorant powder has superior usability (no graininess), deodorizing effects, and anti-discoloration properties.

### Example 2-6

### [Deodorant powder spray]

| | |
|---|---|
| (Powder portion) | |
| Dried alum (average particle size 5 micrometers) | 4. 0 mass % |
| Chlorhydroxy aluminum | 2.0 |
| Zeolite containing silver ions, copper ions and | |
| ammonium ions (average particle size is approximately | |
| 1.5 micrometers; 0.5% or less have a particle size | |
| over 15 micrometers.) | 1.0 |
| Talc | 0. 5 |
| (Oil components) | |
| Decamethylcyclopentasiloxane | 1.5 |
| Perfume | 0.2 |
| (Propellant) | |
| Isopentane | 10.0 |
| Liquefied petroleum gas | 80.8 |

(Preparation method) The powder portion is mixed with a kneader and the oil components are mixed with a blender; a spray can is filled with each of these one after another, and then filled with the propellant to obtain a powder spray.

The obtained powder spray has good dispersibility in the propellant and exhibit no clogging of the nozzle while spraying; it is also superior in terms of the deodorizing effect and anti-discoloration properties.

### Example 2-7

### [Powder spray]

| | |
|---|---|
| (Powder portion) | |
| Alum (average particle size 20 micrometers) | 3.0 mass % |
| Zeolite containing silver ions, zinc ions and | |
| ammonium ions (average particle size is approximately | |
| 5 micrometers; 1% or less have a particle size over | |
| 15 micrometers.) | 2.0 |
| Zinc oxide | 0.2 |
| Silica | 1.5 |
| (Oil components) | |
| Polyoxyethylene nonylphenyl ether | 0.5 |
| Dimethyl polysiloxane (20 mPa·s, 25°C) | 0. 1 |
| Isopropyl myristate | 0.5 |
| (Additives) | |
| Polyoxyethylene sorbitan monooleate | 0.1 |
| Perfume | 0. 1 |
| (Propellant) | |
| Liquefied petroleum gas | 92.0 |

(Preparation method) The powder portion is mixed with a kneader and the oil components are mixed with a blender, to which the additives are added; a spray can is filled with each of these one after another, and then filled with the propellant to obtain a powder spray.

The obtained powder spray has good dispersibility in the propellant and exhibit no clogging of the nozzle while spraying; it is also superior in terms of the deodorizing effect and anti-discoloration properties.

### Example 2-8

### [Compact type deodorant powder]

| | |
|---|---|
| (Powder portion) | |
| Dried alum (average particle size 0.5 micrometers) | 10.0 mass % |
| Zeolite containing copper ions, zinc ions and | |
| ammonium ions (average particle size is approximately | |
| 1.5 micrometers; 0.1% or less have a particle size | |
| over 15 micrometers.) | 10.0 |
| Talc | 60.0 |
| (Oil components) | |
| Methylphenyl polysiloxane (13 mPa·s, 25°C) | 10. 0 |
| Liquid petrolatum | 10.0 |

(Preparation method) The powder portion is mixed with a Henschel mixer; the oil components are added to this mixture, which is then crushed with a 5HP pulverizer (from Hosokawa Micron Ltd.) and molded in a medium plate to obtain a compact type deodorant cosmetic.

The obtained compact type deodorant powder has superior usability (no graininess), deodorizing effects, and anti-discoloration properties.

### Example 2-9

### [Deodorizing spray]

| | |
|---|---|
| (Powder portion) | |
| Alum (average particle size 10 micrometers) | 1.0 mass % |
| Zeolite containing zinc ions and ammonium ions | |
| (average particle size is approximately 5 | |
| micrometers; 5% or less have a particle size over 15 | |
| micrometers.) | 3.0 |
| Zinc oxide | 2.0 |
| (Oil components) | |
| Decamethylcyclopentasiloxane | 5.0 |
| (Additives) | |
| Isopropyl myristate | 0.5 |
| Diglycerol sorbitan tetra-2-ethylhexanoate | 0.5 |
| (Propellant) | 0.5 |
| n-butane | 75.0 |
| i-butane | 13.0 |

(Preparation method) The powder portion is mixed with a kneader and the oil components and the additives are mixed with a blender; a spray can is filled with each of these one after another, and then filled with the propellant to obtain a deodorizing spray.

The obtained deodorizing spray is superior in terms of dispersibility of the powder in the propellant, and has superior usability (no graininess), deodorizing effects, and anti-discoloration properties.

### Example 2-10

### [Baby powder]

| | |
|---|---|
| (Powder portion) | |
| Dried alum (average particle size 50 micrometers) | 15.0 mass % |
| Talc | 65.3 |
| Calcium carbonate | 17.0 |
| Zeolite containing silver ions and ammonium ions | |
| (average particle size is approximately 8 | |
| micrometers; 1% or less have a particle size over 15 | |
| micrometers.) | 2.0 |
| (Oil components) | |
| Methylphenyl polysiloxane (13 mPa·s, 25°C) | 0.4 |
| Dimethyl polysiloxane/polyethylene glycol copolymer | 0.1 |
| (Additives) | |
| Preservative | 0.2 |

(Preparation method) The aforementioned ingredients are thoroughly stirred and mixed to obtain baby powder.

The obtained baby powder has superior usability (no graininess), deodorizing effects, and anti-discoloration properties.

### Example 2-11

| [Deodorant stick] | |
|---|---|
| Methyl trimeticone | 60.0 mass % |
| Squalane | 10.0 |
| Hydrocarbon wax | 10.0 |
| Alum (average particle size 0.05 micrometers) | 5. 0 |
| Zeolite containing zinc ions and ammonium ions | |
| (average particle size is approximately 10 | |
| micrometers; 20% or less have a particle size over 15 | |
| micrometers.) | 15.0 |

(Preparation method) The aforementioned ingredients are mixed and a container is filled with the mixture to obtain a deodorant stick.

The obtained deodorant stick, when applied to armpits, exhibits superior usability (no graininess), deodorizing effects, and anti-discoloration properties.

### Example 2-12

| [Roll-on deodorizing agent] | |
|---|---|
| Dodecamethylcyclohexasiloxane | 67.0 mass % |
| Ethanol | 20.0 |
| Sorbit | 4.0 |
| Dried alum (average particle size 1 micrometer) | 1. 0 |
| Aluminum chloride | 1.0 |
| Magnesium oxide | 2.0 |
| Zeolite containing silver ions, copper ions and | |
| ammonium ions (average particle size is approximately | |
| 2 micrometers; 3% or less have a particle size over | |
| 15 micrometers.) | 5.0 |

(Preparation method) The aforementioned ingredients are mixed and to into a roll-on container to obtain a roll-on deodorizing cosmetic.

The obtained roll-on deodorizing cosmetic exhibits no aggregation of the powder and has superior usability (no graininess), deodorizing effects, and anti-discoloration properties.

### Example 2-13

### [Powder spray]

| | |
|---|---|
| (Powder portion) | |
| Alum (average particle size 0.01 micrometers) | 2.0 mass % |
| Chlorhydroxy aluminum | 0.5 |
| Zeolite containing zinc ions, copper ions and | |
| ammonium ions (average particle size is approximately | |
| 1.5 micrometers; 0.5% or less have a particle size | |
| over 15 micrometers.) | 1.0 |
| Talc | 0.5 |
| (Oil components) | |
| Decamethylcyclopentasiloxane | 1.5 |
| Perfume | 0.2 |
| (Propellant) | |
| Isopentane | 10.0 |
| Liquefied petroleum gas | 83.3 |

(Preparation method) The powder portion is mixed with a kneader and the oil components are mixed with a blender; a spray can is filled with each of these one after another, and then filled with the propellant to obtain a powder spray.

The obtained powder spray has good dispersibility in the propellant and exhibit no clogging of the nozzle while spraying; it spreads well on the skin and is also superior in terms of anti-perspiration properties, the deodorizing effect and anti-discoloration properties.

### Example 2-14

### [Powder spray]

| | |
|---|---|
| (Powder portion) | |
| Dried alum (average particle size 5 micrometers) | 0.5 mass % |
| Zeolite containing silver ions, copper ions and | |
| ammonium ions (average particle size is approximately | |
| 1.0 micrometers; 0.05% or less have a particle size | |
| over 15 micrometers.) | 1.5 |
| Zinc oxide | 0.2 |
| Silica | 1.5 |
| (Oil components) | |
| Polyoxyethylene nonylphenyl ether | 0.5 |
| Dimethyl polysiloxane (1.5 mPa·s, 25°C) | 0.1 |
| Isopropyl myristate | 0.5 |
| (Additives) | |
| Polyoxyethylene sorbitan monooleate | 0.1 |
| Perfume | 0. 1 |
| (Propellant) | |
| Liquefied petroleum gas | 95.0 |

(Preparation method) The powder portion is mixed with a kneader and the oil components are mixed with a blender; a spray can is filled with each of these one after another, and then filled with the propellant to obtain a powder spray.

The obtained powder spray does not show aggregation of the powder components even after being stored for a long time and exhibits good usability as well as sufficient deodorizing effects and anti-discoloration properties.

### Example 2-15

### [Compact type deodorant powder]

| | |
|---|---|
| (Powder portion) | |
| Alum (average particle size 15 micrometers) | 30.0 mass % |
| Zeolite containing silver ions, zinc ions and | |
| ammonium ions (average particle size is approximately | |
| 1.5 micrometers; 1% or less have a particle size over | |
| 15 micrometers.) | 20.0 |
| Talc | 30.0 |
| (Oil components) | |
| Methylphenyl polysiloxane (13 mPa·s, 25°C) | 10. 0 |
| Liquid petrolatum | 10.0 |

(Preparation method) The powder portion is mixed with a Henschel mixer; the oil components are added to this mixture, which is then crushed with a 5HP pulverizer (from Hosokawa Micron Ltd.) and molded in a medium plate to obtain a compact type deodorant cosmetic.

The obtained pressed powder type deodorant cosmetic does not exhibit caking during use, gives a good tactile sensations during use, and has sufficient deodorizing effects and anti-discoloration properties.

### Example 2-16

### [Deodorizing spray]

| | |
|---|---|
| (Propellant) | |
| n-butane | 76.0 mass % |
| i-butane | 15.0 |
| (Oil components) | |
| Dimethyl 1 polysiloxane (1. 5 mPa·s, 25°C) | 5. 0 |
| (Powder portion) | |
| Dried alum (average particle size 4.5 micrometers) | |
| | 2. 5 |
| Zeolite containing zinc ions and ammonium ions | |
| (average particle size is approximately | 0.5 |
| micrometers; 1% or less have a particle size over 15 | |
| micrometers.) | 0.5 |
| (Additives) | |
| Isopropyl myristate | 0.5 |
| Diglycerol sorbitan tetra-2-ethylhexanoate | 0. 5 |

(Preparation method) The powder portion is mixed with a kneader and the oil components and the additives are mixed with a blender; a spray can is filled with each of these one after another, and then filled with the propellant to obtain a deodorizing spray.

The obtained deodorizing spray exhibits good dispersibility of the powder portion in the propellant, gives nice smooth tactile sensations, and exhibits sufficient deodorizing effects and anti-discoloration properties.

### Example 2-17

### [Baby powder]

| | |
|---|---|
| (Powder portion) | |
| Talc | 55.0 mass % |
| Alum (average particle size 25 micrometers) | 25. 0 |
| Calcium carbonate | 17.0 |
| Zeolite containing silver ions, copper ions, and | |
| ammonium ions (average particle size is approximately | |
| 8 micrometers; 20% or less have a particle size over | |
| 15 micrometers.) | 2.3 |
| (Oil components) | |
| Methylphenyl polysiloxane (13 mPa·s, 25°C) | 0. 4 |
| Dimethyl polysiloxane/polyethylene glycol copolymer | 0. 1 |
| (Additives) | |
| Preservative | 0.2 |

(Preparation method) The aforementioned ingredients are thoroughly stirred and mixed to obtain baby powder.

The obtained baby powder does not aggregate, gives smooth sensations during use, and exhibits superior deodorizing effects and anti-discoloration properties.

### Example 2-18

| [Deodorant stick] | |
|---|---|
| Methyl trimeticone | 60.0 mass % |
| Squalane | 10.0 |
| Hydrocarbon wax | 10.0 |
| Zeolite containing silver ions, copper ions and | |
| ammonium ions (average particle size is approximately | |
| 1.5 micrometers; 1.5% or less have a particle size | |
| over 15 micrometers.) | 10.0 |
| Dried alum (average particle size 7 micrometer) | 9. 0 |
| Aluminum/zirconium hydroxychloride | 1.0 |

(Preparation method) The aforementioned ingredients are mixed and a container is filled with the mixture to obtain a deodorant stick.

The obtained deodorant stick, when applied to armpits, gives nice smooth tactile sensations and exhibits superior deodorizing effects and anti-discoloration properties.

### Example 2-19

| [Roll-on deodorizing cosmetic] | |
|---|---|
| Dodecamethylcyclohexasiloxane | 51.0 mass % |
| Ethanol | 20.0 |
| Sorbit | 4.0 |
| Zeolite containing copper ions and ammonium ions | |
| (average particle size is approximately 10 | |
| micrometers; 10% or less have a particle size over 15 | |
| micrometers.) | 5.0 |
| Alum (average particle size 32 micrometers) | 15. 0 |
| Aluminum/zirconium hydroxychloride | 5.0 |

(Preparation method) The aforementioned ingredients are mixed and put into a roll-on container to obtain a roll-on deodorizing cosmetic.

The obtained roll-on deodorizing cosmetic exhibits no aggregation of the powder portion, gives refreshing sensation during use and smoothes the skin, and has superior deodorizing effects and anti-discoloration properties.

### Example 2-20

| [Body cleanser] | |
|---|---|
| Triethanolamine N-lauryl-L-glutamate | 6.0 mass % |
| Sodium N-lauryl methyl taurate | 3.0 |
| Triethanolamine laurate | 9.5 |
| Triethanolamine myristate | 9.5 |
| Lauryl imidazolinium betaine | 5.0 |
| Lauryl diethanol amide | 5.0 |
| Propylene glycol | 7.0 |
| Zeolite containing silver ions and ammonium ions | |
| (average particle size is approximately 5 | |
| micrometers; 5% or less have a particle size over 15 | |
| micrometers.) | 0.5 |
| Dried alum (average particle size 0.01 micrometers) | 1. 0 |
| Aluminum chloride | 0.3 |
| Chlorhydroxy aluminum | 0.2 |
| Methylphenyl polysiloxane (13 mPa·s, 25°C) | 1. 0 |
| Purified water | Balance |
| Perfume | 0.01 |
| Preservative | 0.1 |
| Sodium ethylenediaminetetraacetate | 0.01 |

(Preparation method) Purified water is heated up to 70°C and other ingredients were added one after another and stirred and dissolved. The mixture is cooled down to the ordinary temperature and put into a resin bottle containing stirring balls to obtain a body cleanser.

The obtained body cleanser has cleaning power and foaming power, while maintaining good system stability and usability (no graininess) as well as superior deodorizing effects and anti-discoloration properties.

### Example 2-21

| [Carmine lotion] | |
|---|---|
| Ethanol | 12.5 mass % |
| (Oil components) | |
| Methyl trimeticone | 2.0 |
| (Humectant) | |
| Glycerin | 2.0 |
| 1,3-butylene glycol | 2.0 |
| (Powder agent) | |
| Iron oxide (red iron oxide) | 0.15 |
| Zinc oxide | 0.5 |
| Zeolite containing silver ions and ammonium ions | |
| (average particle size is approximately 1.5 | |
| micrometers; 0. 2% or less have a particle size over | |
| 15 micrometers.) | 0.5 |
| Alum (average particle size 36 micrometers) | 0. 5 |
| Kaolin | 1.5 |
| (Drugs) | |
| Camphor | 0.2 |
| Phenol | 0.02 |
| Perfume | 0.01 |
| Anti-fading agent | 0.01 |
| Purified water | Balance |

(Preparation method) The perfume was added to ethanol, the humectant, and the oil components and dissolved. Camphor and phenol were dissolved in purified water, to which the powder agent, anti-fading agent, and the aforementioned ethanol humectant phase were added and stirred to wet-disperse the powder agent. Filtration was done with approximately 160 mesh to obtain carmine lotion.

The aforementioned carmine lotion has the effect of reducing the burning sensation after sun exposure and is superior in terms of usability with no graininess, deodorizing effects and anti-discoloration properties.

### Example 2-22

### [Essence oil]

| | |
|---|---|
| (Oil components) | |
| Olive oil | 39.69 mass % |
| Liquid petrolatum | 25.0 |
| Squalane | 20.0 |
| (Powder) | |
| Dimethyl polysiloxane (6 mPa·s, 25°C) | 3.0 |
| Zeolite containing silver ions, zinc ions and | |
| ammonium ions (average particle size is approximately | |
| 5 micrometers; 20% or less have a particle size over | |
| 15 micrometers.) | 2.0 |
| Dried alum (average particle size 3 micrometer) | 10. 0 |
| (Others) | |
| Vitamin E acetate | 0.2 |
| Antioxidant | 0.1 |
| Perfume | 0.01 |

(Preparation method) The oil obtained by adding the powder drugs, antioxidant, and perfume to the oil components is put into a resin bottle containing stirring balls to obtain essence oil.

The aforementioned essence oil has good usability (no graininess) and is superior in terms of deodorizing effects and anti-discoloration properties.

### Example 2-23

### [Facial wash]

| | |
|---|---|
| (Fatty acid) | |
| Stearic acid | 10.0 mass % |
| Palmitic acid | 10.0 |
| Myristic acid | 10.0 |
| Lauric acid | 4.0 |
| (Oil components) | |
| Methylphenyl polysiloxane (13 mPa·s, 25°C) | 2. 0 |
| (Alkali) | |
| Potassium hydroxide | 6.0 |
| (Humectant) | |
| PEG 1500 | 10.0 |
| Glycerin | 15.0 |
| (Surfactant) | |
| Glyceryl monostearate | 2.0 |
| POE(20) sorbitan monostearate | 2.0 |
| (Powder) | |
| Zeolite containing silver ions and ammonium ions | |
| (average particle size is approximately 10 | |
| micrometers; 20% or less have a particle size over 15 | |
| micrometers.) | 2.0 |
| Alum (average particle size 9 micrometers) | 2. 0 |
| Preservative | 0.1 |
| Sodium ethylenediaminetetraacetate | 0.05 |
| Perfume | 0.01 |
| Purified water | Balance |

(Preparation method) The fatty acid, oil components, humectant, and preservative are heated and dissolved; the temperature is maintained at 70°C. The purified water, in which the alkali is already dissolved, is added to the oil phase while stirring. After the addition, the temperature is maintained at 70°C to complete the neutralization reaction. The surfactant, chelating agent, perfume, and perfume are dissolved and added; after stirring and mixing, deaeration, and filtration, the mixture is cooled to obtain the facial wash.

The aforementioned facial wash has superior cleaning power and foaming power as well as good usability without graininess; it also has superior deodorizing effects and anti-discoloration properties.

### Example 2-24

### [Facial mask (peel-off type)]

| | |
|---|---|
| (Film agent) | |
| Polyvinyl acetate emulsion | 15.0 mass % |
| Polyvinyl alcohol | 10.0 |
| (Humectant) | |
| Sorbitol | 5.0 |
| PEG 400 | 5.0 |
| (Oil components) | |
| Jojoba oil | 2.0 |
| Methylphenyl polysiloxane (13 mPa·s, 25°C) | 1. 0 |
| Squalane | 1.0 |
| (Surfactant) | |
| POE sorbitan monostearate | 1.0 |
| (Powder) | |
| Titanium oxide | 4.0 |
| Zeolite containing silver ions and ammonium ions | |
| (average particle size is approximately 1.5 | |
| micrometers; 2% or less have a particle size over 15 | |
| micrometers.) | 3.0 |
| Dried alum (average particle size 45 micrometer) | 4. 0 |
| Talc | 4.0 |
| (Alcohol) | |
| Ethanol | 8.0 |
| Perfume | 0.01 |
| Preservative | 0.1 |
| Purified water | Balance |

(Preparation method) The powder is added to the purified water and thoroughly dispersed, to which the humectant is added; after heating up to 70-80°C, the film agent is added and dissolved. The perfume, preservative, surfactant, and oil components are added to the ethanol. This is added to the aforementioned water phase and mixed. After deaeration, filtration, and cooling, a facial mask is obtained.

The aforementioned facial mask has superior usability (no graininess), deodorizing effects, and anti-discoloration properties.

### Example 2-25

### [Pressed powder]

| | |
|---|---|
| (Powder) | |
| Alum (average particle size 0.4 micrometers) | 50.0 mass % |
| Chlorhydroxy aluminum | 1.0 |
| Zeolite containing silver ions, zinc ions and | |
| ammonium ions (average particle size is approximately | |
| 6 micrometers; 15% or less have a particle size over | |
| 15 micrometers.) | 5.0 |
| Talc | 37.0 |
| (Oil components) | |
| Liquid petrolatum | 2.0 |
| Methylphenyl polysiloxane (13 mPa·s, 25°C) | 1.0 |
| Perfume | Appropriate amount |

(Preparation method) After thoroughly mixing the powder components, the perfume, dissolved in the oil components, is uniformly sprayed and mixed. This powder is crushed and then pressure molded to obtain pressed powder.

The aforementioned pressed powder has superior usability (no graininess), deodorizing effects, and anti-discoloration properties.

### Example 2-26

| [Soup] | |
|---|---|
| Sodium lauryl monoglyceride sulfate | Balance |
| Sodium laurylsulfate | 10.0 mass % |
| Sodium cocoate | 30.0 |
| Cetyl alcohol | 3.5 |
| Methylphenyl 1 polysiloxane (13 mPa·s, 25°C) | 0. 5 |
| Zeolite containing silver ions and ammonium ions | |
| (average particle size is approximately 1.5 | |
| micrometers; 1% or less have a particle size over 15 | |
| micrometers.) | 1.0 |
| Dried alum (average particle size 5 micrometer) | 4. 0 |
| Perfume | 0.01 |
| Dye | 0. 01 |
| Antioxidant | 0.1 |
| Sodium ethylenediaminetetraacetate | 0.01 |

(Preparation method) The aforementioned ingredients are put into a mixer for mixing and stirring, and then kneaded and compressed with a roll and plotter; the mixture is then shaped into a bar and extruded and molded to obtain soap.

The aforementioned soap has superior usability (no graininess), deodorizing effects, and anti-discoloration properties.

### Example 2-27

### [Emollient lotion]

| | |
|---|---|
| (Oil components) | |
| Cetyl alcohol | 1.0 mass % |
| Beeswax | 0.5 |
| Petrolatum | 2.0 |
| Squalane | 6.0 |
| Dimethyl polysiloxane (1.5 mPa·s, 25°C) | 2. 0 |
| (Alcohol) | |
| Ethanol | 5.0 |
| (Humectant) | |
| Glycerin | 4.0 |
| 1,3-butylene glycol | 4.0 |
| (Surfactant) | |
| POE (10) monooleic ester | 1.0 |
| Glyceryl monostearate | 1.0 |
| (Viscous fluid) | |
| Quince seed extract (5% aqueous solution) | 20. 0 |
| (Powder) | |
| Zeolite containing silver ions, zinc ions and | |
| ammonium ions (average particle size is approximately | |
| 3. 5 micrometers; 5% or less have a particle size over | |
| 15 micrometers.) | 2.0 |
| Alum (average particle size 12 micrometers) | 1. 0 |
| Phenoxyethanol | 0.05 |
| Coloring agent | 0.01 |
| Perfume | 0.01 |
| Purified water | Balance |

(Preparation method) The humectant and coloring agent are added to the purified water and the temperature is raised and adjusted to 70°C . The surfactant and preservative are added to the oil components and the temperature is raised and adjusted to 70°C. This is added to the aforementioned water phase to carry out preliminary emulsification. The quince seed extract, powder , and ethanol 1 are added to this, followed by stirring; after homogenizing the emulsified particles using a homomixer, the mixture is deaerated, filtered, and cooled to obtain an emollient lotion.

The aforementioned emollient lotion has superior usability (no graininess), deodorizing effects, and anti-discoloration properties.

### Example 2-28

### [Oil-based gel (emulsified type)]

| | |
|---|---|
| (Oil components) | |
| Liquid petrolatum | 10.0 mass % |
| Glycerol tri-2-ethylhexanoate | 48.0 |
| Decamethylcyclopentasiloxane | 2.0 |
| (Humectant) | |
| Sorbitol | 10.0 |
| PEG 400 | 5.0 |
| (Surfactant) | |
| Sodium lauroylmethyltaurate | 5.0 |
| POE octyldodecyl alcohol ether | 10.0 |
| (Powder) | |
| Zeolite containing silver ions, zinc ions and | |
| ammonium ions (average particle size is approximately | |
| 2. 0 micrometers; 3% or less have a particle size over | |
| 15 micrometers.) | 2.0 |
| Dried alum (average particle size 18 micrometer) | 2. 0 |
| Perfume | 0.01 |
| Purified water | Balance |

(Preparation method) The humectant and acylmethyltaurine are added to the purified water and the temperature is raised and adjusted to 70°C. POE octyldodecyl ether and perfume are added to the oil components and the temperature is raised and adjusted to 70°C. This and the powder are gradually added to the aforementioned water phase. After homogenizing the emulsified particles using a homomixer, the mixture is deaerated, filtered, and cooled to obtain an oil based gel.

The aforementioned oil based gel has superior usability (no graininess), deodorizing effects, and anti-discoloration properties.

### Example 2-29

### [Cream]

| | |
|---|---|
| (Oil components) | |
| Cetyl alcohol | 5.0 mass % |
| Stearic acid | 3.0 |
| Methylphenyl polysiloxane (13 mPa·s, 25°C) | 1.0 |
| Petrolatum | 4.0 |
| Squalane | 9.0 |
| Glycerol tri-2-ethylhexanoate | 7.0 |
| (Humectant) | |
| Dipropylene glycol | 5.0 |
| Glycerin | 5.0 |
| (Surfactant) | |
| Propylene glycol monostearate | 3.0 |
| POE (20) cetyl alcohol ether | 3.0 |
| (Alkali) | |
| Triethanolamine | 1.0 |
| (Powder) | |
| Zeolite containing silver ions and ammonium ions | |
| (average particle size is approximately 1.5 | |
| micrometers; 1.5% or less have a particle size over | |
| 15 micrometers.) | 1.0 |
| Alum (average particle size 0.9 micrometers) | 0.1 |
| Preservative | 0.1 |
| Antioxidant | 0.05 |
| Perfume | 0.01 |
| Purified water | Balance |

(Preparation method) The humectant and alkali are added to the purified water and the temperature is raised and adjusted to 70°C. The oil components are heated and dissolved, to which the surfactant, preservative, antioxidant, and perfume are added and the temperature is adjusted to 70°C. This is added to the aforementioned water phase to carry out preliminary emulsification. The powder is added and a homomixer is used to homogenize the emulsified particles, followed by deaeration, filtration, and cooling.

The aforementioned cream has superior usability (no graininess), deodorizing effects, and anti-discoloration properties.

### Example 2-30

| [Wet sheet] | |
|---|---|
| Ion-exchanged water | 65.38 mass % |
| Ethanol | 30.00 |
| Zeolite containing silver ions, zinc ions, and | |
| ammonium ions (average particle size is approximately | |
| 3 micrometers; 1% or less have a particle size over | |
| 15 micrometers.) | 1.0 |
| Alum (average particle size 0.09 micrometers) | |
| Polyoxyethylene polyoxypropylene decyltetradecyl | 3. 0 |
| ether | 0.4 |
| Citric acid | 0.04 |
| Sodium citrate | 0.04 |
| Adenine | 0.05 |
| Trisodium ethylenediaminehydroxyethyl triacetate | 0.05 |
| Camphor | 0.01 |
| Menthol | 0.03 |

(Preparation method) The water soluble ingredients are thoroughly dissolved in the ion-exchanged water, to which the insoluble ingredients are added; the insoluble ingredients are well dispersed and at the same time non-woven fabric is soaked in the mixture and then put into an aluminum pouch pack.

The aforementioned wet sheet has superior usability (no graininess), deodorizing effects, and anti-discoloration properties.

### Example 2-31

| [Powder in puff] | |
|---|---|
| Talc | 69.66 mass % |
| Polymethylsilsesquioxane spherical powder | |
| | 10. 0 |
| Spherical calcium alginate powder | 3.0 |
| Zeolite containing silver ions, zinc ions, and | |
| ammonium ions (average particle size is approximately | |
| 10 micrometers; 18% or less have a particle size over | |
| 15 micrometers.) | 2.0 |
| Dried alum (average particle size 6 micrometers) | 5. 0 |
| Ethylparaben | 0.1 |
| Salicylic acid | 0.2 |
| Fine particle zinc oxide (average particle size 60 nm) | |
| | 5. 0 |
| Zinc oxide-coated spherical polyethylene powder | 5. 0 |
| Iron oxide (yellow) | 0.015 |
| Iron oxide (red) | 0.025 |

(Preparation method) The aforementioned ingredients are thoroughly mixed with a Henschel mixer, and pulverized by a pulverizer; a non-woven bag is filled with this and then put into a puff.

The aforementioned powder in puff has superior usability (no graininess), deodorizing effects, and anti-discoloration properties.

### Example 2-32

| [Deodorant stick (wax type)] | |
|---|---|
| Decamethylcyclopentasiloxane | 0.1 mass % |
| Dimethyl polysiloxane (1.5 mPa·s, 25°C) | 10. 0 |
| Stearyl alcohol | 8.0 |
| Polyoxypropylene (40) butyl ether | 7.0 |
| Sorbitan sesquiisostearate | 2.5 |
| Hydrogenated castor oil | 1.5 |
| Alum (average particle size 0.15 micrometers) | 20. 0 |
| Zeolite containing silver ions, zinc ions, and | |
| ammonium ions (average particle size is approximately | |
| 3. 0 micrometers; 10% or less have a particle size over | |
| 15 micrometers.) | 18.0 |
| Talc | 10. 7 |
| Fine particle zinc oxide (average particle size 60 nm) | 1. 0 |
| Hydroxypropyl- *β* -cyclodextrin | 0.1 |
| Zinc oxide-coated spherical nylon 12 | |
| | 1. 0 |
| Disodium calcium ethylenediaminetetraacetate | 0. 1 |

(Preparation method) The oil components are heated, melted, and thoroughly mixed, to which the powder ingredients are added; the mixture is then homogeneously dispersed and mixed with a homomixer while being heated, and then poured into a mold and cooled to obtain a stick.

The aforementioned deodorant stick has superior usability (no graininess), deodorizing effects, and anti-discoloration properties.

### Example 2-33

| [Deodorant stick (non-oil type)] | |
|---|---|
| Talc | 49.0 mass % |
| Sericite | 20.0 |
| (Dimeticone/vinyl dimeticone) cross polymer | |
| spherical powder | 5.0 |
| Dried alum (average particle size 4 micrometers) | 10. 0 |
| Polymethylsilsesquioxane spherical powder | 5. 0 |
| Zeolite containing silver ions, zinc ions, and | |
| ammonium ions (average particle size is approximately | |
| 0.9 micrometers; 0.3% or less have a particle size | |
| over 15 micrometers.) | 10.0 |
| Aluminum magnesium silicate | 1.0 |

(Preparation method) One weight-part of aluminum magnesium silicate and 20 parts of ion-exchanged water are mixed to obtain gel ; the other ingredients are thoroughly mixed and dispersed into it to obtain slurry, which is poured into a mold, put into a dryer to evaporate the moisture, and then cooled to obtain a stick.

The aforementioned deodorant stick has superior usability (no graininess), deodorizing effects, and anti-discoloration properties.

### Example 2-34

| [Water based gel] | |
|---|---|
| POE (14) POP (7) dimethyl ether | 7.0 mass % |
| PEG 1500 | 8.0 |
| Zeolite containing silver ions, copper ions, and | |
| ammonium ions (average particle size is approximately | |
| 5 micrometers; 20% or less have a particle size over | |
| 15 micrometers.) | 1.0 |
| Alum (average particle size 10 micrometers) | 3. 0 |
| Carboxyvinyl polymer | 0.4 |
| Methylcellulose | 0.2 |
| P0E (15) oleyl alcohol ether | 1.0 |
| Potassium hydroxide | 0.1 |
| ε -polylysine | 0. 2 |
| Tetrasodium edetate | 0.05 |
| Perfume | 0.1 |
| Purified water | 78.95 |

(Preparation method) The water soluble polymer is homogeneously dissolved in the purified water and then ε-polylysine and tetrasodium edetate are dissolved. The surfactant is added to the POE (14) P0P (7) dimethyl ether and heated/dissolved, to which the perfume is added. The previously prepared water phase is gradually added, and finally the potassium hydroxide aqueous solution is added and thoroughly stirred for neutralization.

The aforementioned water based gel has superior usability (no graininess), deodorizing effects, and anti-discoloration properties.

### Example 2-35

| | |
|---|---|
| [Medicated body cleanser] | |
| Triethanolamine lauryl sulfate (40% aqueous | |
| solution) | 40.0 mass % |
| Sodium lauryl polyoxyethylene (3 mole) sulfate (30% | |
| aqueous solution) | 20.0 |
| Lauryl diethanolamide | 5.0 |
| Zeolite containing silver ions, zinc ions, and | |
| ammonium ions (average particle size is approximately | |
| 3.5 micrometers; 1% or less have a particle size over | |
| 15 micrometers.) | 2.0 |
| Alum (average particle size 42 | micrometers) |
| | 2. 0 |
| Glycerol palmitate | 1.0 |
| Lanolin derivative | 2.0 |
| Propylene glycol | 5.0 |
| Purified water | Balance |
| Perfume | Appropriate amount |
| Dye | Appropriate amount |
| Trisodium ethylenediaminehydroxyethyl triacetate | |
| (dihydrate salt) | Appropriate amount |

(Preparation method) The water soluble ingredients are thoroughly mixed, to which the powder ingredients are added and thoroughly mixed and dispersed; the mixture is then put into a container. The container is shaken well before use.

The aforementioned body cleanser has superior usability (no graininess), deodorizing effects, and anti-discoloration properties.

### [Invention of claims 7-8]

Recipes shown in Table 3-1 and Table 3-2 were used to prepare powder lotion-type antiperspirant lotions and a prescribed amount (4 micrograms/cm²) is applied on clothing. The clothing was exposed to sunlight (10 minutes) and washed with common laundry detergent in a washing machine; the degree of staining was then evaluated by visual observation. For Comparative examples, recipes not containing polyoxyethylene polyoxypropylene 2-decyltetradecyl ether (20-28 E. 0. ) (10-16 P. 0. ) and recipes containing polyoxyethylene (E. 0. 60) were investigated. The degree of staining was evaluated by giving O to those that are within the acceptable range for skin treatment compositions, and X to those that are outside of this range. 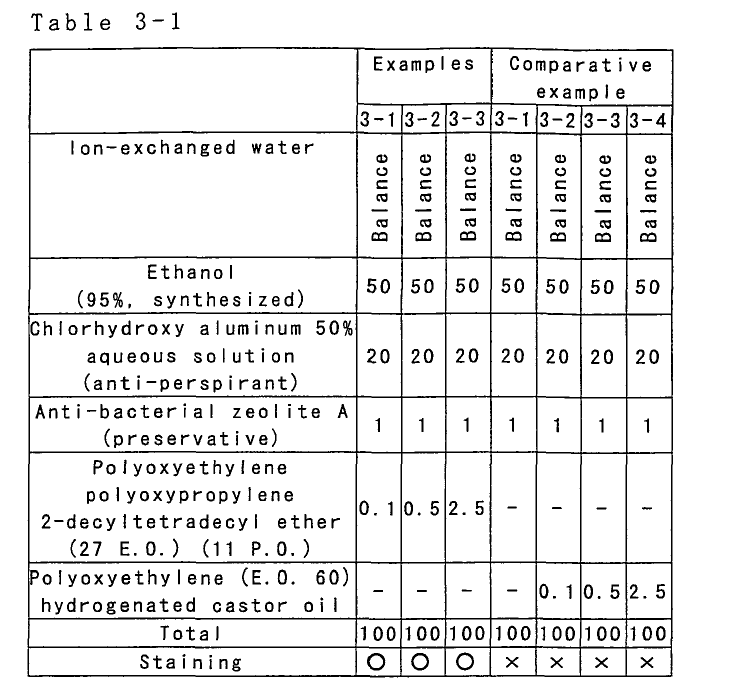 Anti-bacterial zeolite A: Zeolite containing silver ions and zinc ions (average particle size approximately 1.5 micrometers)

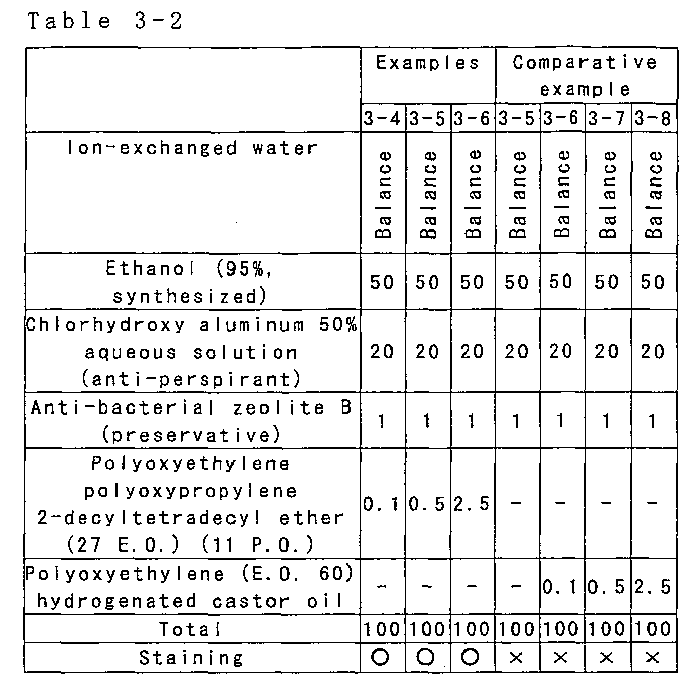

Anti-bacterial zeolite B: Zeolite containing silver ions, zinc ions, and ammonium ions (Zeomic AJ10N from Sinanen Zeomic Co., Ltd., average particle size approximately 1.5 micrometers)

The aforementioned results indicate that staining is significant on Comparative examples not containing polyoxyethylene polyoxypropylene 2-decyltetradecyl ether (20-28 E. 0. ) (10-16 P. 0. ) and Comparative examples containing polyoxyethylene (E. 0. 60) instead of polyoxyethylene polyoxypropylene 2-decyltetradecyl ether (20-28 E. 0. ) (10-16 P. 0. ) .

On the other hand, Examples containing polyoxyethylene polyoxypropylene 2-decyltetradecyl ether (20-28 E. 0. ) (10-16 P. 0. ) show very little staining, which is within the allowable range for skin treatment compositions; this indicates superior anti-staining effects.

Other Examples of the present invention are shown below.

### Example 3-7: Pressed powder

| | |
|---|---|
| Chlorhydroxy aluminum | 5 mass % |
| Zinc oxide (zinc flower) | 5 |
| Talc | Balance |
| Liquid petrolatum | 3 |
| Anti-bacterial zeolite B | 10 |
| Polyoxyethylene polyoxypropylene 2-decyltetradecyl | |
| ether (20 E. 0. ) (16 P.0.) | 0.01 |

### Example 3-8: Loose powder

| | |
|---|---|
| Chlorhydroxy aluminum | 5 mass % |
| Zinc oxide (zinc flower) | 5 |
| Talc | Balance |
| Anti-bacterial zeolite B | 10 |
| Polyoxyethylene polyoxypropylene 2-decyltetradecyl | |
| ether (22 E.0.) (15 P.0.) | 0.05 |

### Example 3-9: Lotion-type spray

| | |
|---|---|
| (Stock solution recipe) | |
| Purified water | 10 mass % |
| Chlorhydroxy aluminum | 10 |
| Anhydrous ethyl alcohol | Balance |
| Isopropyl myristate | 2 |
| 1,3-butylene glycol | 3 |
| Anti-bacterial zeolite B | 1 |
| Polyoxyethylene polyoxypropylene 2-decyltetradecyl | |
| ether (26 E. 0. ) (13 P. 0. ) | 1 |
| (Filler recipe) | |
| Stock solution | 50 |
| LPG | 50 |

### Example 3-10: Powder spray

| | |
|---|---|
| Chlorhydroxy aluminum | 20 mass % |
| Silicic acid anhydride | 15 |
| Talc | 20 |
| Zinc oxide (zinc flower) | 5 |
| Isopropyl myristate | Balance |
| Dimethyl polysiloxane | 10 |
| Sorbitan fatty acid ester | 3 |
| Anti-bacterial zeolite B | 5 |
| Polyoxyethylene polyoxypropylene 2-decyltetradecyl | |
| ether (24 E.0.) (12 P.0.) | 0.1 |
| (Filler recipe) | |
| Stock solution | 10 |
| LPG | 90 |

### Example 3-11: Powder spray

| | |
|---|---|
| Alum | 20 mass % |
| Silicic acid anhydride | 15 |
| Talc | 20 |
| Zinc oxide (zinc flower) | 5 |
| Isopropyl myristate | Balance |
| Polyoxyethylene/polyoxypropylene random polymer | |
| methyl ether | 10 |
| Sorbitan fatty acid ester | 3 |
| Anti-bacterial zeolite B | 5 |
| Polyoxyethylene polyoxypropylene 2-decyltetradecyl | |
| ether (27 E.0.) (11 P.0.) | 0. 1 |
| (Filler recipe) | |
| Stock solution | 10 |
| LPG | 90 |

### Example 3-12: Stick

| | |
|---|---|
| Chlorhydroxy aluminum | 20 mass % |
| Talc | 8 |
| Zinc oxide (zinc flower) | 5 |
| Solid petrolatum wax | 2 |
| Stearyl alcohol | 8 |
| Liquid petrolatum | 15 |
| Cyclic dimethyl polysiloxane | Balance |
| Sorbitan fatty acid ester | 1 |
| Anti-bacterial zeolite B | 5 |
| Polyoxyethylene polyoxypropylene 2-decyltetradecyl | |
| ether (22 E.0.) (15 P.0.) | 4 |

### Example 3-13: Cream

| | |
|---|---|
| Purified water Balance | |
| Squalane | 20 mass % |
| Cyclic dimethyl polysiloxane | 15 |
| Glyceryl diisostearate | 3 |
| Diethoxyethyl succinate | 5 |
| Organically modified montmorillonite | 1.5 5 |
| 1,3-butylene glycol | 5 |
| Anti-bacterial zeolite A | 5 |
| Polyoxyethylene polyoxypropylene 2-decyltetradecyl | |
| ether (24 E.0.) (13 P.0.) | 2 |

### Example 3-14: Emulsion

| | |
|---|---|
| Purified water | Balance |
| Chlorhydroxy aluminum | 20 mass % |
| 0ctyl-p-methoxycinnamate | 5 |
| 4-tert butyl-4'-methoxybenzoylmethane | 1 |
| Hydrophobically treated zinc oxide | 5 |
| Polyoxyethylene/polyoxypropylene random polymer | |
| methyl ether | 10 |
| Silicone oil | 15 |
| Silicone resin | 1 |
| Glyceryl diisostearate | 1 |
| Organically modified montmorillonite | 0.5 |
| 1,3-butylene glycol | 5. 5 |
| Anti-bacterial zeolite B | 13 |
| Polyoxyethylene polyoxypropylene 2-decyltetradecyl | |
| ether (25 E.0.) (11 P.0.) | 1 |

### Example 3-15: Ointment

| | |
|---|---|
| Purified water | Balance |
| Chlorhydroxy aluminum | 20 mass % |
| Glycerin | 10 |
| 1,3-butylene glycol | 3 |
| Caustic potash | 0.25 |
| Stearic acid | 2 |
| Stearic acid monoglyceride | 2 |
| Cetanol | 1 |
| Liquid petrolatum | 5 |
| Petrolatum | 2 |
| Anti-bacterial zeolite B | 1 |
| Polyoxyethylene polyoxypropylene 2-decyltetradecyl | |
| ether (20 E.0.) (16 P.0.) | 2.5 |

### Example 3-16: Gel

| | |
|---|---|
| Purified water | Balance |
| Chlorhydroxy aluminum | 20 mass % |
| Dipropylene glycol | 5 |
| PEG 1500 | 5.5 |
| Carboxyvinyl polymer | 0.4 |
| Methylcellulose | 0.2 |
| POE (15) oleyl alcohol ether | 0.5 |
| Potassium hydroxide | 0.1 |
| EDTA | 0.02 |
| Anti-bacterial zeolite B | 5 |
| Polyoxyethylene polyoxypropylene 2-decyltetradecyl | |
| ether (26 E.0.) (12 P.0.) | 0.3 |

### Example 3-17: Wet sheet

| | |
|---|---|
| Purified water | Balance |
| Anhydrous ethyl alcohol | 35 mass % |
| Polyoxyethylene hydrogenated castor | 0.1 |
| Citric acid (food) | 0.02 |
| Sodium citrate | 0.06 |
| Anti-bacterial zeolite B | 2 |
| Polyoxyethylene polyoxypropylene 2-decyltetradecyl | |
| ether (26 E.0.) (12 P.0.) | 0. 01 |

### INDUSTRIAL APPLICABILITY

(1) The present invention can provide a skin treatment composition containing anti-bacterial zeolite that exhibits the effect of preventing discoloration of the skin treatment compositions and/or reducing the degree of discoloration.
(2) The present invention can provide a skin treatment composition that is a deodorizing cosmetic containing anti-bacterial zeolite and also is superior in formulation stability such as anti-discoloring properties and dispersibility of powder components, as well as very superior in terms of the tactile sensation during use. The skin treatment composition of the present invention has superior usability (no graininess) because dispersibility of the powder ingredients such as anti-bacterial zeolite, alum, and dried alum is superior and the powder does not aggregate. It also has superior anti-discoloration properties.
(3) The present invention can provide a skin treatment composition containing anti-bacterial zeolite that exhibits the effect of preventing staining of clothing due to adhesion of the skin treatment composition and/or reducing the degree of such staining.

The present invention is a skin treatment composition containing anti-bacterial zeolite that exhibits the effect of preventing staining of clothing due to adhesion of the skin treatment composition and/or reducing the degree of such staining.

## Claims

1. A skin treatment composition comprising anti-bacterial zeolite and polyoxyethylene polyoxypropylene 2-decyltetradecyl ether.

2. The skin treatment composition of claim 1 wherein the polyoxyethylene unit of the polyoxyethylene polyoxypropylene 2-decyltetradecyl ether is 20-28 E.O. and the polyoxypropylene unit is 10-16 P.O.
